(19) Europäisches Patentamt
European Patent Office
Office européen des brevets

(11) **EP 3 849 518 B1**

(12) **EUROPEAN PATENT SPECIFICATION**

(45) Date of publication and mention
of the grant of the patent:
**18.03.2026 Bulletin 2026/12**

(21) Application number: **19765736.4**

(22) Date of filing: **12.09.2019**

(51) International Patent Classification (IPC):
*A61K 9/08* (2006.01)     *A61K 9/19* (2006.01)
*A61K 39/395* (2006.01)   *A61P 35/00* (2006.01)
*A61P 19/02* (2006.01)    *C07K 16/28* (2006.01)
*A61K 45/06* (2006.01)    *A61K 47/26* (2006.01)

(52) Cooperative Patent Classification (CPC):
(C-Sets available)
**A61K 9/08; A61K 9/19; A61K 39/39591;
A61K 45/06; A61K 47/26; A61P 19/02;
A61P 35/00; C07K 16/2866;** C07K 2317/24;
C07K 2317/76                            (Cont.)

(86) International application number:
**PCT/EP2019/074303**

(87) International publication number:
**WO 2020/053321 (19.03.2020 Gazette 2020/12)**

(54) **CSF-1R ANTIBODY FORMULATION**

CSF-1R-ANTIKÖRPERFORMULIERUNG

FORMULATIONS D'ANTICORPS CSF-1R

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.09.2018 EP 18194145**

(43) Date of publication of application:
**21.07.2021 Bulletin 2021/29**

(73) Proprietor: **F. Hoffmann-La Roche AG
4070 Basel (CH)**

(72) Inventors:
• **RAVURI, Satya, Krishna, Kishore
4070 Basel (CH)**
• **YANG, Kewei
4070 Basel (CH)**

(74) Representative: **Klostermeyer-Rauber, Dörte
F. Hoffmann-La Roche AG
Patent Department
Grenzacherstrasse 124
4070 Basel (CH)**

(56) References cited:
**WO-A1-2011/070024     WO-A1-2016/106180
WO-A1-2016/128318**

Remarks:
The complete document including Reference
Table(s) and the Sequence Listing(s) can be
downloaded from the EPO website

(52) Cooperative Patent Classification (CPC): (Cont.)

C-Sets
**A61K 39/39591, A61K 2300/00**

## Description

### Field of the Invention

The present invention relates to a formulation of an antibody molecule against CSF-1R comprising a heavy chain variable region comprising the amino acid sequence of SEQ ID NO: 7 and a light chain variable region comprising the amino acid sequence of SEQ ID NO:8, a process for the preparation of said formulation and uses of the formulation.

### Background

The human CSF-1 receptor (CSF-1R; colony stimulating factor 1 receptor; synonyms: M-CSF receptor; Macrophage colony-stimulating factor 1 receptor, Fms proto-oncogene, c-fms, SEQ ID NO: 13) is known since 1986 (Coussens, L., et al., Nature 320 (1986) 277-280). CSF-1R is the receptor for CSF-1 (colony stimulating factor 1, also called M-CSF, macrophage colony-stimulating factor) and mediates the biological effects of this cytokine (Sherr, C.J., et al., Cell 41 (1985) 665-676). The cloning of the colony stimulating factor-1 receptor (CSF-1R) (also called c-fms) was described for the first time in Roussel, M.F., et al., Nature 325 (1987) 549-552. In that publication, it was shown that CSF-1R had transforming potential dependent on changes in the C-terminal tail of the protein including the loss of the inhibitory tyrosine 969 phosphorylation which binds Cbl and thereby regulates receptor down regulation (Lee, P.S., et al., Embo J. 18 (1999) 3616-3628). A second ligand for CSF-1R termed interleukin-34 (IL-34) was also identified (Lin, H., et al, Science 320 (2008) 807-811).

Colony-stimulating factor 1 (CSF-1) and its receptor, CSF-1R, regulate the migration, differentiation, and survival of macrophages and their precursors. CSF-1R is a member of the receptor protein tyrosine kinase (rPTK) family of growth factor receptors, which includes several known proto-oncogenes. Diffuse-type tenosynovial giant cell tumor (TGCT) of the soft tissue (alternatively known as pigmented villonodular synovitis [PVNS]), a rare proliferative disease affecting large joints, is characterized by an overexpression of CSF-1. In the majority of TGCT patients, chromosomal translocations involving the gene encoding CSF-1 result in overexpression of this cytokine by cells within the synovial lining. This leads to massive recruitment of CSF-1R-expressing cells, mainly nonmalignant mononuclear and multinucleated cells that form the bulk tumorous mass. Marginal excision or complete synovectomy remain the treatments of choice for TGCT, but the disorder sometimes necessitates mutilating surgery due to locally destructive and recurring tumor growth. Emactuzumab, an antibody against CSF-1R, has been shown to be successful in treating this rare disease (Cassier, P., et al., Lancet Oncol. 16 (2015) 949-956).

The main biological effects of CSF-1R signaling are the differentiation, proliferation, migration, and survival of hematopoietic precursor cells to the macrophage lineage (including osteoclast). Activation of CSF-1R is mediated by its ligands, CSF-1 (M-CSF) and IL-34. Binding of CSF-1 (M-CSF) to CSF-1R induces the formation of homodimers and activation of the kinase by tyrosine phosphorylation (Li, W. et al, EMBO Journal. 10 (1991) 277-288; Stanley, E.R., et al., Mol. Reprod. Dev. 46 (1997) 4-10).

The biologically active homodimer CSF-1 binds to the CSF-1R within the subdomains D1 to D3 of the extracellular domain of the CSF-1 receptor (CSF-1R-ECD). The CSF-1R-ECD comprises five immunoglobulin-like subdomains (designated Dl to D5). The subdomains D4 to D5 of the extracellular domain (CSF-1R-ECD) are not involved in the CSF-1 binding. (Wang, Z., et al Molecular and Cellular Biology 13 (1993) 5348-5359). The subdomain D4 is involved in dimerization (Yeung, Y-G., et al Molecular & Cellular Proteomics 2 (2003) 1143-1155; Pixley, F. J., et al., Trends Cell Biol 14 (2004) 628-638). Antibodies that bind to human CSF-1R fragment delD4 of SEQ ID NO:11 (a human CSF-1R fragment in which the D4 subdomain of human CSF-1R-ECD was deleted) are described in WO 2011/070024 A1. These antibodies block the receptor dimerization interface with their epitope being located within D4 and D5 and are therefore unique. One of these antibodies is Emactuzumab or RG7155. Its CDR and VH/VL sequences are disclosed herein.

Antibody molecules, as part of the group of protein pharmaceuticals, are very susceptible to physical and chemical degradation. Chemical degradation includes any process that involves modification of the protein via bond formation or cleavage, yielding a new chemical entity. A variety of chemical reactions is known to affect proteins. These reactions can involve hydrolysis including cleavage of peptide bonds as well as deamidation, isomerization, oxidation and decomposition. Physical degradation refers to changes in the higher order structure and includes denaturation, adsorption to surfaces, aggregation and precipitation. Protein stability is influenced by the characteristics of the protein itself, e.g. the amino acid sequence, the glycosylation pattern, and by external influences, such as temperature, solvent pH, excipients, interfaces, or shear rates. So, it is important to define the optimal formulation conditions to protect the protein against degradation reactions during manufacturing, storage and administration. (Manning, M. C., et al. (1989), "Stability of protein pharmaceuticals", Pharm Res 6(11), 903-918; Zheng, J. Y., Janis, L. J. (2005), "Influence of pH, buffer species, and storage temperature on physicochemical stability of a humanized monoclonal antibody LA298", Int. J. Pharmaceutics 308, 46-51). Stable liquid formulations of therapeutic antibodies are particularly difficult to obtain when the formulation should include antibodies in a high concentration.

WO 2016/128318 A1 discloses pharmaceutical formulations for antibodies, in particular CSF-1R antibodies, comprising glycine and sucrose. WO 2016/106180 A1 describes the use of CSF-1R antibodies for the treatment of pigmented villonodular synovitis (PVNS) and WO 2011/070024 A1 discloses the CSF-1R antibody as described herein.

It is an object of the present invention to provide a stable formulation for the anti-CSF-1R antibody as described herein with as few as necessary excipients, which enables the desired dosing and allows convenient administration of the antibody to a patient.

The formulation of the present invention shows good stability upon storage for 24 months at the intended storage temperature of 2 to 8 °C without formation of visible particles that will allow i.v. administration without the need of an in-line filter allowing greater administration convenience. Shaking and multiple freezing-thawing steps were applied to the liquid formulation to simulate physical stress conditions that potentially occur during manufacturing or transportation of the drug product. The formulation of the present invention shows good stability after applying shaking and freeze-thaw stress.

## Summary

The present invention relates to a stable, high-dose pharmaceutical formulation of an antibody which binds to CSF-1R comprising a heavy chain variable region comprising the amino acid sequence of SEQ ID NO: 7 and a light chain variable region comprising the amino acid sequence of SEQ ID NO:8, a process for the preparation of the formulation and uses of the formulation.

In one aspect, the invention refers to a pharmaceutical formulation comprising:

- 40 mg/ml to 200 mg/ml of an antibody against CSF-1R comprising a heavy chain variable region comprising the amino acid sequence of SEQ ID NO: 7 and a light chain variable region comprising the amino acid sequence of SEQ ID NO:8;

- 0.01 % w/v to 0.1% w/v of a surfactant;

- 5 mM to 100 mM of a buffering agent;

- 10 mM to 500 mM of at least one stabilizer;

at a pH in the range from 4.5 to 7.0.

The formulation according to the invention may be provided in liquid form, lyophilized form or in liquid form reconstituted from a lyophilized form.

In a preferred aspect, the concentration of the antibody against CSF-1R comprised in the formulation according to the invention is in the range of 40 to 100 mg/ml, preferably 40 to 75 mg/ml, more preferably 40 to 60 mg/ml. Particularly preferred is a concentration of 50 mg/ml.

In another aspect, the pharmaceutical formulation comprises a surfactant in a concentration range of from 0.01% w/v to 0.1 % w/v. In the formulation of the invention, the concentration of the surfactant is described as a percentage, expressed in weight/volume (w/v). Preferably, the pharmaceutical formulation comprises a surfactant in a concentration range of 0.02% w/v to about 0.05% w/v, most preferably of 0.04 % w/v. The pharmaceutical formulation according to claims 1 or 2, wherein the surfactant is a polysorbate. Preferred surfactants for use in the present invention are polyoxyethylen-sorbitan fatty acid esters (i.e. polysorbates), preferably polysorbate 20 or polysorbate 80. In a particular aspect, the surfactant is polysorbate 20.

In a further aspect, the pharmaceutical formulation according to the invention comprises a buffering agent. Preferably, the buffering agent is a histidine buffer. Histidine buffers are buffers having histidine, generally L-histidine, as buffering agent. Most preferred is L-histidine/HCl buffer, comprising L-histidine or mixtures of L-histidine and L-histidine hydrochloride and pH adjustment achieved with hydrochloric acid. Unless otherwise indicated, the term "histidine" when used herein to describe a buffering agent, refers to L-histidine/HCl buffer, particularly a histidine chloride buffer. In one aspect, the buffering agent has a concentration in the range of 10 to 30 mM, more particularly of 20 mM.

Preferably, the pH of the formulation is in the range of 5.0 to 6.5, particularly at about 6.0. Thus, the pH of the formulation is preferably 6.0. Regardless of the buffering agent used, the pH can be adjusted with an acid or a base known in the art, e.g. hydrochloric acid, acetic acid, phosphoric acid, sulfuric acid and citric acid, sodium hydroxide and potassium hydroxide.

In another aspect, the pharmaceutical formulation according to the invention comprises at least one stabilizer is selected from group consisting of salts, saccharides and amino acids. In a particular aspect, the at least one stabilizer is a saccharide, in particular an oligosaccharide selected from the group consisting of sucrose, trehalose, lactose, maltose and raffinose. More particularly, the saccharide is sucrose. In a preferred aspect, the stabilizer or the saccharide is present in a concentration in the range from 140 to 250 mM, particularly in the range from 210 to 230 mM. Preferably, the saccharide is present in a concentration of 220 mM.

In yet another aspect, the pharmaceutical formulation according to the invention comprises a first stabilizer selected from the group of salts, saccharides and amino acids, and methionine as a second stabilizer. In preferred aspect, the first stabilizer is present in a concentration of 120 to 300 mM, and the second stabilizer methionine is present in a concentration of 5 to 25 mM. More preferably, methionine is present in a concentration of 10 mM.

The antibody against CSF-1R comprised in the formulation of the present invention is preferably an antibody that binds to human CSF-1R fragment delD4 (SEQ ID NO: 11) and to human CSF-1R extracellular Domain (SEQ ID NO:12) with a ratio of 1:50 or lower.

In one particular aspect, the invention relates to a pharmaceutical formulation, which comprises

40 to 100 mg/ml of an antibody against CSF-1R;
20 mM L-histidine;
0.03% w/v to 0.05% w/v polysorbate 20;
210 to 230 mM sucrose;
optionally 5 to 25 mM methionine;
at a pH of 6.0 $\pm$ 0.5.

In one particular aspect, the pharmaceutical formulation according to the invention comprises

50 mg/ml of an antibody against CSF-1R;
20 mM L-histidine;
0.04% w/v polysorbate 20;
220 mM sucrose;
10 mM methionine;
at a pH of 6.0 $\pm$ 0.5.

In a further aspect, the invention relates to the pharmaceutical formulation as described herein before for use in the treatment of cancer or metastasisIn a preferred aspect, the pharmaceutical formulation as described herein before is for use in the treatment of pigmented villonodular synovitis (PVNS) or tenosynovial giant cell tumors (TGCT).

In another aspect, the pharmaceutical formulation as described herein before is for use in combination with another therapeutic agent, in particular another immunotherapy. In one particular aspect, the pharmaceutical formulation is for use in combination with an agent blocking PD-L1/PD-1 interaction.

**Detailed Description of the Invention**

The present invention relates to a stable pharmaceutical formulation comprising an antibody against CSF-1R.

The term "pharmaceutical formulation" or "pharmaceutical composition" refers to preparations which are in such form as to permit the biological activity of the active ingredients to be unequivocally effective, and which contain no additional components which are toxic to the subjects to which the formulation is administered.

The term "liquid" as used herein in connection with the formulation according to the invention denotes a formulation which is liquid at a temperature of at least about 2 °C to about 8 °C under atmospheric pressure.

The term "lyophilized" as used herein in connection with the formulation according to the invention denotes a formulation which is manufactured by freeze-drying methods known in the art *per se.* The solvent (e.g. water) is removed by freezing followed by sublimation of the ice under vacuum and desorption of residual water at elevated temperature. The lyophilizate usually has a residual moisture of about 0.1 to 5% (w/w) and is present as a powder or a physically stable cake. The lyophilizate is characterized by a fast dissolution after addition of a reconstitution medium.

The term "reconstituted form" as used herein in connection with the formulation according to the invention denotes a formulation which is lyophilized and re-dissolved by addition of reconstitution medium. Suitable reconstitution media comprise but are not limited to water for injection (WFI), bacteriostatic water for injection (BWFI), sodium chloride solutions (e.g. 0.9% (w/v) NaCl), glucose solutions (e.g. 5% glucose), surfactant-containing solutions (e.g. 0.01% polysorbate 20), pH-buffered solutions (e.g. phosphate-buffered solutions).

The formulation according to the invention is physiologically well tolerated, can be prepared easily, can be dispensed precisely and is stable with respect to decomposition products and aggregates over the duration of storage, during repeated freezing and thawing cycles and mechanical stress.

A "stable" formulation is one in which the protein therein, e.g. the antibody, essentially retains its physical and chemical stability and thus its biological activity upon storage.

A "stable liquid pharmaceutical antibody formulation" is a liquid antibody formulation with no significant changes observed at a refrigerated temperature (2-8 °C) for at least 12 months, particularly 2 years, and more particularly 3 years. The criteria for stability are the following: no more than 10%, particularly 5%, of antibody monomer is degraded as measured by size exclusion chromatography (SEC-HPLC). Furthermore, the solution is colorless or clear to slightly opalescent by visual analysis. The protein concentration of the formulation has no more than +/- 10% change. No more than 10%, particularly 5% of aggregation is formed. The stability is measured by methods known in the art such UV spectroscopy, size exclusion chromatography (SEC-HPLC), Ion-Exchange Chromatography (IE-HPLC), turbidimetry and visual inspection.

The term "antibody" encompasses the various forms of antibody structures including but not being limited to whole antibodies and antibody fragments. The antibody according to the invention is in particular a human antibody, a humanized antibody, chimeric antibody, antibody fragment, or further genetically engineered antibody as long as the characteristic properties according to the invention are retained. More particularly, the antibody is a humanized monoclonal antibody, especially a recombinant humanized antibody. In a particular aspect, the humanized antibody is of the human IgG1 isotype.

The term "humanized antibody" refers to antibodies in which the framework or "complementarity determining regions" (CDR) have been modified to comprise the CDR of an immunoglobulin of different specificity as compared to that of the parent immunoglobulin. In a preferred embodiment, a murine CDR is grafted into the framework region of a human antibody to prepare the "humanized antibody." See e.g. Riechmann, L., et al., Nature 332 (1988) 323-327; and Neuberger, M.S., et al., Nature 314 (1985) 268-270. Particularly preferred CDRs correspond to those representing sequences recognizing the antigens noted above for chimeric antibodies. Other forms of "humanized antibodies" encompassed by the present invention are those in which the constant region has been additionally modified or changed from that of the original antibody to generate the properties according to the invention, especially in regard to C1q binding and/or Fc receptor (FcR) binding.

An "antibody against CSF-1R" is an antibody that specifically binds to human CSF-1R. Particularly useful antibodies against CSF-1R are the antibodies described e.g. in PCT publication WO 2011/070024 A1. These antibodies are unique in that they bind to human CSF-1R fragment delD4 (comprising the extracellular subdomains D1 -D3 and D5, SEQ ID NO:11) and to human CSF-1R Extracellular Domain (CSF-1R-ECD) (comprising the extracellular subdomains D1 -D5, SEQ ID NO:12) with a ratio of 1:50 or lower. With its epitope thus located within D4 and D5 they are able to block the receptor dimerization interface.

By "specific binding" is meant that the binding is selective for the antigen and can be discriminated from unwanted or non-specific interactions. The ability of an antigen binding moiety to bind to a specific antigenic determinant can be measured either through an enzyme-linked immunosorbent assay (ELISA) or other techniques familiar to one of skill in the art, e.g. surface plasmon resonance (SPR) technique (analyzed e.g. on a BIAcore instrument) (Liljeblad et al., Glyco J 17, 323-329 (2000)), and traditional binding assays (Heeley, Endocr Res 28, 217-229 (2002)). In one embodiment, the extent of binding of an antigen binding moiety to an unrelated protein is less than about 10% of the binding of the antigen binding moiety to the antigen as measured, e.g., by SPR. In certain embodiments, an antigen binding moiety that binds to the antigen, or an antibody comprising that antigen binding moiety, has a dissociation constant ($K_D$) of $\leq 1\ \mu M$, $\leq 100\ nM$, $\leq 10$ nM, $\leq 1\ nM$, $\leq 0.1\ nM$, $\leq 0.01\ nM$, or $\leq 0.001\ nM$ (e.g. $10^{-8}$ M or less, e.g. from $10^{-8}$ M to $10^{-13}$ M, e.g., from $10^{-9}$ M to $10^{-13}$ M).

In a particular aspect, the antibody against CSF-1R comprises a heavy chain variable region comprising the heavy chain CDR1 (CDR-H1) of SEQ ID NO: 1, the CDR-H2 of SEQ ID NO: 2, and the CDR-H3 of SEQ ID NO: 3; and a light chain variable region comprising the light chain CDR1 (CDR-L1) of SEQ ID NO: 4, the CDR-L2 of SEQ ID NO: 5 and the CDR-L3 of SEQ ID NO: 6. More particularly, the antibody against CSF-1R comprises a heavy chain variable region comprising the amino acid sequence of SEQ ID NO: 7 and a light chain variable region comprising the amino acid sequence of SEQ ID NO:8. In a particular preferred aspect, the antibody against CSF-1R is a human IgG1 antibody and comprises heavy chains comprising the amino acid sequence of SEQ ID NO:9 and light chains comprising the amino acid sequence of SEQ ID NO:10. This antibody is called emactuzumab or RG7155.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three complementary determining regions (CDRs). A single VH or VL domain may be sufficient to confer antigen-binding specificity. As used herein in connection with variable region sequences, "Kabat numbering" refers to the numbering system set forth by Kabat et al., Sequences of Proteins of Immunological Interest, 5th Ed. Public Health Service, National Institutes of Health, Bethesda, MD (1991).

The term "hypervariable region" or "HVR" as used herein refers to each of the regions of an antibody variable domain which are hypervariable in sequence and which determine antigen binding specificity. HVRs comprise the amino acid residues from "complementarity determining regions" ("CDRs"). Generally, antibodies comprise six CDRs: three in the VH (CDR-H1, CDR-H2, CDR-H3), and three in the VL (CDR-L1, CDR-L2, CDR-L3). Exemplary CDRs herein include:

(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991)); and

(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)).

Unless otherwise indicated, the CDRs are determined according to Kabat et al., *supra.* One of skill in the art will understand that the CDR designations can also be determined according to Chotia, supra, McCallum, supra, or any other scientifically accepted nomenclature system.

"Framework" or "FR" refers to variable domain residues other than complementary determining regions (CDRs). The FR of a variable domain generally consists of four FR domains: FR1, FR2, FR3, and FR4. Accordingly, the CDR and FR sequences generally appear in the following sequence in VH (or VL): FR1-CDR-H1(L1)-FR2-CDR-H2(L2)-FR3-CDR-H3(L3)-FR4.

The term "epitope" includes any polypeptide determinant capable of specific binding to an antibody. In certain embodiments, epitope determinant include chemically active surface groupings of molecules such as amino acids, sugar side chains, phosphoryl, or sulfonyl, and, in certain embodiments, may have specific three dimensional structural characteristics, and or specific charge characteristics. An epitope is a region of an antigen that is bound by an antibody.

The "constant regions" or "constant domains" are not involved directly in binding an antibody to an antigen, but exhibit various effector functions. Depending on the amino acid sequence of the constant region of their heavy chains, antibodies or immunoglobulins are divided in the classes: IgA, IgD, IgE, IgG and IgM, and several of these may be further divided into subclasses (isotypes), e.g. IgG1, IgG2, IgG3 and IgG4, IgA1 and IgA2. The antibodies used in the invention are particularly of IgG type, more particularly of IgG1 or IgG4 human subtype.

The term "Fc region" herein is used to define a C-terminal region of an immunoglobulin heavy chain that contains at least a portion of the constant region. The term includes native sequence Fc regions and variant Fc regions. In one aspect, a human IgG heavy chain Fc region extends from Cys226, or from Pro230, to the carboxyl-terminus of the heavy chain. However, antibodies produced by host cells may undergo post-translational cleavage of one or more, particularly one or two, amino acids from the C-terminus of the heavy chain. Therefore an antibody produced by a host cell by expression of a specific nucleic acid molecule encoding a full-length heavy chain may include the full-length heavy chain, or it may include a cleaved variant of the full-length heavy chain. This may be the case where the final two C-terminal amino acids of the heavy chain are glycine (G446) and lysine (K447, numbering according to Kabat EU index). Therefore, the C-terminal lysine (Lys447), or the C-terminal glycine (Gly446) and lysine (Lys447), of the Fc region may or may not be present. Amino acid sequences of heavy chains including an Fc region are denoted herein without C-terminal glycine-lysine dipeptide if not indicated otherwise. In one aspect, a heavy chain including an Fc region as specified herein, comprised in an antibody according to the invention, comprises an additional C-terminal glycine-lysine dipeptide (G446 and K447, numbering according to EU index of Kabat). In one aspect, a heavy chain including an Fc region as specified herein, comprised in an antibody according to the invention, comprises an additional C-terminal glycine residue (G446, numbering according to EU index of Kabat). Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

The concentration of the antibody against CSF-1R comprised in the pharmaceutical formulation is in the range of 40 mg/ml to 200 mg/ml, particularly in the range of 40 mg/ml to 100 mg/ml, more particularly in the range of 40 mg/ml to 60 mg/ml and most particularly of 50 mg/ml.

The term "surfactant" as used herein denotes a pharmaceutically acceptable, surface-active agent. Preferably, a non-ionic surfactant is used. Examples of pharmaceutically acceptable surfactants include, but are not limited to, polyoxyethylen-sorbitan fatty acid esters (Tween), polyoxyethylene alkyl ethers (Brij), alkylphenylpolyoxyethylene ethers (Triton X), polyoxyethylene-polyoxypropylene copolymers (Poloxamer, Pluronic), and sodium dodecyl sulphate (SDS). Preferred polyoxyethylene-sorbitan fatty acid esters are polysorbate 20 (polyoxyethylene sorbitan monolaureate, sold under the trademark Tween 20™) and polysorbate 80 (polyoxyethylene sorbitan monooleate, sold under the trademark Tween 80™). Preferred polyethylene-polypropylene copolymers are those sold under the names Pluronic® F68 or Poloxamer 188™. Preferred polyoxyethylene alkyl ethers are those sold under the trademark Brij™. Preferred alkylphenylpolyoxyethylene ethers are sold under the tradename Triton X, most preferred is p-tert-octylphenoxy polyethoxyethanol (sold under the tradename Triton X-100™). Preferred surfactants for use in the present invention are polyoxyethylen-sorbitan fatty acid esters, preferably polysorbate 20 or polysorbate 80, most preferably polysorbate 20. Another preferred surfactant is Poloxamer 188™.

The term "buffering agent" as used herein denotes a pharmaceutically acceptable excipient, which stabilizes the pH of a pharmaceutical preparation. Suitable buffers are well known in the art and can be found in the literature. Preferred pharmaceutically acceptable buffers comprise but are not limited to histidine-buffers, citrate-buffers, succinate-buffers, acetate-buffers, arginine-buffers, phosphate-buffers or mixtures thereof. Buffering agents are thus histidine salts, citrate salts, succinate salts, acetate salts, malate salts, phosphate salts and lactate salts. Buffering agents of particular interest comprise L-histidine or mixtures of L-histidine and L-histidine hydrochloride or L-histidine acetate with pH adjustment with an acid or a base known in the art. The above mentioned buffers are generally used in an amount of about 5 mM to about 100 mM, particularly of about 10 mM to about 30 mM and more particularly of about 20 mM. Independently from the buffer used, the pH can be adjusted to a value in the range from 4.5 to 7.0 and particularly to a value in the range from 5.0 to 6.0 and most particularly to pH 6.0 ± 0.03 with an acid or a base known in the art, e.g. hydrochloric acid, acetic acid, phosphoric acid, sulfuric acid and citric acid, sodium hydroxide and potassium hydroxide.

The term "stabilizer" denotes a pharmaceutical acceptable excipient, which protects the active pharmaceutical ingredient and/or the formulation from chemical and/or physical degradation during manufacturing, storage and application. Stabilizers include but are not limited to saccharides, amino acids, polyols (e.g. mannitol, sorbitol, xylitol, dextran, glycerol, arabitol, propylene glycol, polyethylene glycol), cyclodextrines (e.g. hydroxypropyl-β-cyclodextrine, sulfobutylethyl-β-cyclodextrine, β-cyclodextrine), polyethylenglycols (e.g. PEG 3000, PEG 3350, PEG 4000, PEG 6000), albumines (human serum albumin (HSA), bovine serum albumin (BSA)), salts (e.g. sodium chloride (saline), magnesium chloride, calcium chloride), chelators (e.g. EDTA) as hereafter defined. Stabilizers that are particularly used in the present invention, are selected from the group consisting of saccharides, polyols and amino acids. Stabilizers can be present in the formulation in an amount of about 10 mM to about 500 mM, particularly in an amount of about 140 to about 250 mM and more particularly in an amount of about 210 mM to about 240 mM. More particularly, sucrose or trehalose are used as stabilizers in an amount of about 220 mM to about 240 mM.

The term "saccharide" as used herein includes monosaccharides and oligosaccharides. A monosaccharide is a monomeric carbohydrate which is not hydrolysable by acids, including simple sugars and their derivatives, e.g. aminosugars. Saccharides are usually in their D conformation. Examples of monosaccharides include glucose, fructose, galactose, mannose, sorbose, ribose, deoxyribose, neuraminic acid. An oligosaccharide is a carbohydrate consisting of more than one monomeric saccharide unit connected via glycosidic bond(s) either branched or in a linear chain. The monomeric saccharide units within an oligosaccharide can be identical or different. Depending on the number of monomeric saccharide units the oligosaccharide is a di-, tri-, tetra- pentaand so forth saccharide. In contrast to polysaccharides the monosaccharides and oligosaccharides are water soluble. Examples of oligosaccharides include sucrose, trehalose, lactose, maltose and raffinose. Preferred saccharides for use in the present invention are sucrose and trehalose (i.e. α,α-D-trehalose), most preferred is sucrose. Trehalose is available as trehalose dihydrate. Saccharides can be present in the formulation in an amount of about 10 to about 500 mM, preferably in an amount of about 200 to about 300 mM, more preferably in an amount of about 220 to about 250 mM, particularly an amount of about 220 mM or about 240 mM, most preferably in an amount of about 220 mM.

The term "amino acid" as used herein denotes a pharmaceutically acceptable organic molecule possessing an amino moiety located at α-position to a carboxylic group. Examples of amino acids include but are not limited to arginine, glycine, ornithine, lysine, histidine, glutamic acid, asparagic acid, isoleucine, leucine, alanine, phenylalanine, tyrosine, tryptophane, methionine, serine, proline. The amino acid employed is preferably in each case the L-form. Basic amino acids, such as arginine, histidine, or lysine, are preferably employed in the form of their inorganic salts (advantageously in the form of the hydrochloric acid salts, i.e. as amino acid hydrochlorides). A preferred amino acid for use in the present invention is methionine. Methionine is preferably used at a concentration of about 5 to about 25 mM, most preferably about 10 mM.

A subgroup within the stabilizers are lyoprotectants. The term "lyoprotectant" denotes pharmaceutically acceptable excipients, which protect the labile active ingredient (e.g. a protein) against destabilizing conditions during the lyophilisation process, subsequent storage and reconstitution. Lyoprotectants comprise but are not limited to the group consisting of saccharides, polyols (such as e.g. sugar alcohols) and amino acids. Preferred lyoprotectants can be selected from the group consisting of saccharides such as sucrose, trehalose, lactose, glucose, mannose, maltose, galactose, fructose, sorbose, raffinose, neuraminic acid, amino sugars such as glucosamine, galactosamine, N-methylglucosamine ("Meglumine"), polyols such as mannitol and sorbitol, and amino acids such as arginine and glycine or mixtures thereof. Lyoprotectants are generally used in an amount of about 10 to 500 mM, preferably in an amount of about 10 to about 300 mM and more preferably in an amount of about 100 to about 300 mM.

A further subgroup within the stabilizers are antioxidants. The term "antioxidant" denotes pharmaceutically acceptable excipients, which prevent oxidation of the active pharmaceutical ingredient. Antioxidants comprise but are not limited to ascorbic acid, gluthathione, cysteine, methionine, citric acid, EDTA. Antioxidants can be used in an amount of about 0.01 to about 100 mM, preferably in an amount of about 5 to about 50 mM and more preferably in an amount of about 5 to about 25 mM.

The formulations according to the invention may also comprise one or more tonicity agents. The term "tonicity agents" denotes pharmaceutically acceptable excipients used to modulate the tonicity of the formulation. The formulation can be hypotonic, isotonic or hypertonic. Isotonicity in general relates to the osmotic pressure of a solution, usually relative to that of human blood serum (around 250-350 mOsmol/kg). The formulation according to the invention can be hypotonic, isotonic or hypertonic but will preferably be isotonic. An isotonic formulation is liquid or liquid reconstituted from a solid form, e.g. from a lyophilized form, and denotes a solution having the same tonicity as some other solution with which it is compared, such as physiologic salt solution and the blood serum. Suitable tonicity agents comprise but are not limited to sodium chloride, potassium chloride, glycerine and any component from the group of amino acids or sugars, in particular glucose. Tonicity agents are generally used in an amount of about 5 mM to about 500 mM. Within the stabilizers and tonicity agents there is a group of compounds which can function in both ways, i.e. they can at the same time be a stabilizer and a tonicity agent. Examples thereof can be found in the group of sugars, amino acids, polyols, cyclodextrines, polyethyleneglycols and salts. An example for a sugar which can at the same time be a stabilizer and a tonicity agent is trehalose.

The term "polyols" as used herein denotes pharmaceutically acceptable alcohols with more than one hydroxy group. Suitable polyols comprise to but are not limited to mannitol, sorbitol, glycerine, dextran, glycerol, arabitol, propylene glycol, polyethylene glycol, and combinations thereof. Polyols can be used in an amount of about 10 mM to about 500 mM, particularly in an amount of about 10 to about 250 mM and more particularly in an amount of about 200 to about 250 mM.

The formulations may also contain adjuvants such as preservatives, wetting agents, emulsifying agents and dispersing agents. Prevention of presence of microorganisms may be ensured both by sterilization procedures, and by the inclusion of various antibacterial and antifungal agents, e.g. paraben, chlorobutanol, phenol, sorbic acid, and the like. Preservatives are generally used in an amount of about 0.001 to about 2% (w/v). Preservatives comprise but are not limited to ethanol, benzyl alcohol, phenol, m-cresol, p-chlor-m-cresol, methyl or propyl parabens, benzalkonium chloride.

The pharmaceutical formulation may also contain adjuvants such as preservatives, wetting agents, emulsifying agents and dispersing agents. Prevention of presence of microorganisms may be ensured both by sterilization procedures, and by the inclusion of various antibacterial and antifungal agents, for example, paraben, chlorobutanol, phenol, sorbic acid, and the like. Preservatives are generally used in an amount of about 0.001 to about 2 %(w/v). Preservatives comprise but are not limited to ethanol, benzyl alcohol, phenol, m-cresol, p-chlor-m-cresol, methyl or propyl parabens, benzalkonium chloride.

A formulation of the present invention can be administered by a variety of methods known in the art. As will be appreciated by the skilled artisan, the route and/or mode of administration will vary depending upon the desired results. To administer a formulation of the invention by certain routes of administration, it may be necessary to dilute the formulation in a diluent. Pharmaceutically acceptable diluents include saline, glucose, Ringer and aqueous buffer solutions.

Preferably, the formulation according to the invention is administered by intravenous (IV), subcutaneous (SC), or any other parental administration means such as those known in the pharmaceutical art. In a preferred aspect, the pharmaceutical formulation is administered by IV infusion. When administered via intravenous injection, it may be administered as a bolus injection or as a continuous infusion. For instance, the pharmaceutical formulation of the invention can be diluted with a sterile saline solution and administered with an infusion pump as normally used in clinical setting.

The phrases "parenteral administration" and "administered parenterally" as used herein mean modes of administration other than enteral and topical administration, usually by injection, and include, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

The pharmaceutical formulation according to the invention is suitably administered to the patient at one time or over a series of treatments and may be administered to the patient at any time from diagnosis onwards; it may be administered as the sole treatment or in conjunction with other drugs or therapies useful in treating the conditions as described herein before.

The antibody against CSF-1R may be the sole active ingredient in the liquid pharmaceutical composition. Alternatively, the antibody against CSF-1R may be administered in combination, e.g. simultaneously, sequentially or separately, with one or more other therapeutically active ingredients. The term "active ingredient" as used herein refers to an ingredient with a pharmacological effect, such as a therapeutic effect, at a relevant dose. Accordingly, the antibody against CSF-1R in the liquid pharmaceutical composition may be accompanied by other active ingredients including other antibody ingredients, for example a CD40 antibody, a VEGF antibody or an agent blocking PD-L1/PD-1 interaction. In particular, the agent blocking PD-L1/PD-1 interaction is an anti-PD-L1 antibody or an anti-PD1 antibody. More particularly, the agent blocking PD-L1/PD-1 interaction is selected from the group consisting of atezolizumab, durvalumab, pembrolizumab and nivolumab. In a specific aspect, the agent blocking PD-L1/PD-1 interaction is atezolizumab. In a particular aspect, the CD40 antibody is selicrelumab. In another preferred aspect, the VEGF antibody is bevacizumab (Avastin).

The pharmaceutical compositions suitably comprise a therapeutically effective amount of antibody. The term "therapeutically effective amount" as used herein refers to an amount of a therapeutic agent needed to treat, ameliorate or prevent a targeted disease or condition, or to exhibit a detectable therapeutic, pharmacological or preventative effect. For any antibody, the therapeutically effective amount can be estimated initially either in cell culture assays or in animal models, usually in rodents, rabbits, dogs, pigs or primates. The animal model may also be used to determine the appropriate concentration range and route of administration. Such information can then be used to determine useful doses and routes for administration in humans.

The precise therapeutically effective amount for a human subject will depend upon the severity of the disease state, the general health of the subject, the age, weight and gender of the subject, diet, time and frequency of administration, drug combination(s), reaction sensitivities and tolerance/response to therapy. This amount can be determined by routine experimentation and is within the judgement of the clinician. In a particular aspect, the antibody against CSF-1R is administered at a (fixed) dose of 600-1200 mg, particularly at a dose of 750-1100 mg, more particularly at a dose of 750-1000 mg and even more particularly at a dose of 900-1000 mg. In a preferred aspect, the dose is 1000 mg.

In a preferred aspect, the pharmaceutical formulation is for use in treatment cycles. In particular, the treatment cycles have a length between 2 and 4 weeks, preferably between 18 and 24 days. More preferably, the treatment cycles have a length of (about) 3 weeks.

The pharmaceutical formulation may be conveniently presented in unit dose forms containing a predetermined amount of the antibody against CSF-1R. In a particular aspect, the pharmaceutical formulation will be provided in vials for storage at 2 to 8 °C. In a preferred aspect, the pharmaceutical formulation will be provided in vials of the size of 20 ml. In another preferred aspect, the pharmaceutical formulation will be provided in vials of the size of 50 ml.

The stable formulations to be used for in vivo administration must be sterile. This is readily accomplished by filtration through sterile filtration membranes. The formulation must be fluid to the extent that the formulation is deliverable by syringe or an infusion system. In addition to water, the carrier can be an isotonic buffered saline solution, ethanol, polyol (e.g., glycerol, propylene glycol, and liquid polyethylene glycol, and the like), and suitable mixtures thereof. In view of their high stability the pharmaceutical formulation according to the invention can be administered i.v. without the need of an in-line filter and is thus much more convenient to handle than conventional formulations that need to be administered with an in-line filter. In-line filters such as Sterifix® have to be installed in the infusion line of i.v. medications to prevent the administration of any particles, air, or microorganisms that may be in the i.v. solution or line. Particles of 5 to 20 microns size and larger have the capability of obstructing blood flow through pulmonary capillaries, which could lead to complications such as pulmonary embolism. Foreign particles can also cause phlebitis at the injection site and filters may help to reduce the incidence of phlebitis.

The stable pharmaceutical formulation according to the invention can be prepared by methods known in the art, e.g. ultrafiltration-diafiltration, dialysis, addition and mixing, lyophilisation, reconstitution, and combinations thereof. Examples of preparations of formulations according to the invention can be found herein after.

The invention thus comprises a process for the preparation of the formulations according to the invention. Said process comprises buffer-exchanging the antibody against a diafiltration buffer containing the anticipated buffer composition, and, where required, concentration of the antibody by diafiltration, followed by adding the excipients (e.g., sucrose, sodium chloride, methionine) as stock solutions to the antibody solution, followed by adding the surfactant as stock solution to the antibody/excipient solution, and finally adjusting the antibody concentration to the desired final concentration using buffer solution, whereby also the final excipient and surfactant concentrations are reached.

Alternatively, the excipients can also be added as solids to the starting solution comprising the antibody. If the antibody is in the form of a solid, e.g. a lyophilizate, the formulation according to the invention can be prepared by firstly dissolving the antibody in water or buffer solution, optionally comprising one or more of the excipients, and subsequently adding the further excipients as stock solutions or solids. The antibody can advantageously also be dissolved directly in a solution comprising all further excipients. One or more of the excipients present in the formulation according to the invention may already be added during or at the end of the process for the preparation of the antibody, e.g. by dissolving the antibody directly in a solution comprising one, more than one, or preferably all of the excipients of the formulation in the final step of the purification carried out after the preparation of the antibody. If the solution comprising the antibody and the excipients does not yet have the desired pH, this is adjusted by addition of an acid or base, preferably using the acid or base already present in the buffer system. This is followed by sterile filtration.

The stable liquid pharmaceutical formulations according to the invention can also be in a lyophilized form or in a liquid form reconstituted from the lyophilized form. The "lyophilized form" is manufactured by freeze-drying methods known in the art. The lyophilizate usually has a residual moisture content of about 0.1 to 5% (w/w) and is present as a powder or a physically stable cake. The "reconstituted form" can be obtained from the lyophilizate by a fast dissolution after addition of reconstitution medium. Suitable reconstitution media comprise but are not limited to water for injection (WFI), bacteriostatic water for injection (BWFI), sodium chloride solutions (e.g. 0.9% (w/v) NaCl), glucose solutions (e.g. 5% (w/v) glucose), surfactant-containing solutions (e.g. 0.01% (w/v) polysorbate 20 and pH-buffered solutions (e.g. phosphate-buffered solutions).

The invention further comprises the formulations according to the invention for use in treating diseases, particularly for the treatment of cancer, and in the treatment of pigmented villonodular synovitis (PVNS) or tenosynovial giant cell tumors (TGCT).

The term "cancer" as used herein may be, for example, lung cancer, non-small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma, lymphoma, lymphocytic leukemia, including refractory versions of any of the above cancers, or a combination of one or more of the above cancers. In a preferred aspect, the cancer is a breast cancer, colorectal cancer, melanoma, head and neck cancer, lung cancer or prostate cancer. In another preferred aspect, such cancer is breast cancer, lung cancer, colon cancer, ovarian cancer, melanoma cancer, bladder cancer, renal cancer, kidney cancer, liver cancer, head and neck cancer, colorectal cancer, pancreatic cancer, gastric carcinoma cancer, esophageal cancer, mesothelioma, prostate cancer, leukemia, lymphoma, myelomas. In one preferred aspect, such cancers are further characterized by CSF-1 or CSF-IR expression or overexpression. In another aspect, the pharmaceutical formulation of the present invention is for use in the simultaneous treatment of primary tumors and new metastases. In a further aspect, the pharmaceutical formulation of the invention is for use in the treatment of periodontitis, histiocytosis X, osteoporosis, Paget's disease of bone (PDB), bone loss due to cancer therapy, periprosthetic osteolysis, glucocorticoid-induced osteoporosis, rheumatoid arthritis, psioratic arthritis, osteoarthritis, inflammatory arthridities, and inflammation. In another preferred aspect, the pharmaceutical formulation is for use in melanoma, urinary bladder cancer (UCB), or lung cancer (e.g. non small cell lung (NSCL) cancer). In another preferred aspect, the pharmaceutical formulation is for use in renal cell carcinoma (RCC) or Head and Neck Squamous Cell Carcinoma (HNSCC). In a particularly preferred aspect, the pharmaceutical formulation of the present invention is for use in the treatment of pigmented villonodular synovitis (PVNS) or tenosynovial giant cell tumors (TGCT).

**Table with Sequences:**

| SEQ ID NO: | Name | Sequence |
|---|---|---|
| 1 | CDR-H1 | SYDIS |
| 2 | CDR-H2 | VIWTDGGTNY AQKLQG |
| 3 | CDR-H3 | DQRLYFDV |
| 4 | CDR-L1 | RASEDVNTYV S |
| 5 | CDR-L2 | AASNRYT |
| 6 | CDR-L3 | QQSFSYPT |
| 7 | VH | QVQLVQSGAE VKKPGASVKV SCKASGYTFT SYDISWVRQA PGQGLEWMGV IWTDGGTNYA QKLQGRVTMT TDTSTSTAYM ELRSLRSDDT AVYYCARDQR LYFDVWGQGT TVTVSS |
| 8 | VL | DIQMTQSPSS LSASVGDRVT ITCRASEDVN TYVSWYQQKP GKAPKLLIYA ASNRYTGVPS RFSGSGSGTD FTLTISSLQP EDFATYYCQQ SFSYPTFGQG TKLEIK |

(continued)

| SEQ ID NO: | Name | Sequence |
|---|---|---|
| 9 | Heavy chain | QVQLVQSGAE VKKPGASVKV SCKASGYTFT<br>SYDISWVRQA PGQGLEWMGV IWTDGGTNYA<br>QKLQGRVTMT TDTSTSTAYM ELRSLRSDDT<br>AVYYCARDQR LYFDVWGQGT TVTVSSASTK<br>GPSVFPLAPS SKSTSGGTAA LGCLVKDYFP<br>EPVTVSWNSG ALTSGVHTFP AVLQSSGLYS<br>LSSVVTVPSS SLGTQTYICN VNHKPSNTKV<br>DKKVEPKSCD KTHTCPPCPA PELLGGPSVF<br>LFPPKPKDTL MISRTPEVTC VVVDVSHEDP<br>EVKFNWYVDG VEVHNAKTKP REEQYNSTYR<br>VVSVLTVLHQ DWLNGKEYKC KVSNKALPAP<br>IEKTISKAKG QPREPQVYTL PPSRDELTKN<br>QVSLTCLVKG FYPSDIAVEW ESNGQPENNY<br>KTTPPVLDSD GSFFLYSKLT VDKSRWQQGN<br>VFSCSVMHEA LHNHYTQKSL SLSP |
| 10 | Light chain | DIQMTQSPSS LSASVGDRVT ITCRASEDVN<br>TYVSWYQQKP GKAPKLLIYA ASNRYTGVPS<br>RFSGSGSGTD FTLTISSLQP EDFATYYCQQ<br>SFSYPTFGQG TKLEIKRTVA APSVFIFPPS<br>DEQLKSGTAS VVCLLNNFYP REAKVQWKVD<br><br>NALQSGNSQE SVTEQDSKDS TYSLSSTLTL<br>SKADYEKHKV YACEVTHQGL SSPVTKSFNR GEC |
| 11 | human CSF-1R fragment delD4 | IPVIEPSVPE LVVKPGATVT LRCVGNGSVE<br>WDGPPSPHWT LYSDGSSSIL STNNATFQNT<br>GTYRCTEPGD PLGGSAAIHL YVKDPARPWN<br>VLAQEVVVFE DQDALLPCLL TDPVLEAGVS<br>LVRVRGRPLM RHTNYSFSPW HGFTIHRAKF<br>IQSQDYQCSA LMGGRKVMSI SIRLKVQKVI<br>PGPPALTLVP AELVRIRGEA AQIVCSASSV<br>DVNFDVFLQH NNTKLAIPQQ SDFHNNRYQK<br>VLTLNLDQVD FQHAGNYSCV ASNVQGKHST<br>SMFFRYPPEV SVIWTFINGS GTLLCAASGY<br>PQPNVTWLQC SGHTDRCDEA QVLQVWDDPY<br>PEVLSQEPFH KTVQSLLTVE TLEHNQTYEC<br>RAHNSVGSGS WAFIPISAGA HTHPPDE |

(continued)

| SEQ ID NO: | Name | Sequence |
|---|---|---|
| 12 | human CSF-1R Extracellular Domain | IPVIEPSVPE LVVKPGATVT LRCVGNGSVE WDGPPSPHWT LYSDGSSSIL STNNATFQNT GTYRCTEPGD PLGGSAAIHL YVKDPARPWN VLAQEVVVFE DQDALLPCLL TDPVLEAGVS LVRVRGRPLM RHTNYSFSPW HGFTIHRAKF IQSQDYQCSA LMGGRKVMSI SIRLKVQKVI PGPPALTLVP AELVRIRGEA AQIVCSASSV DVNFDVFLQH NNTKLAIPQQ SDFHNNRYQK VLTLNLDQVD FQHAGNYSCV ASNVQGKHST SMFFRVVESA YLNLSSEQNL IQEVTVGEGL NLKVMVEAYP GLQGFNWTYL GPFSDHQPEP KLANATTKDT YRHTFTLSLP RLKPSEAGRY SFLARNPGGW RALTFELTLR YPPEVSVIWT FINGSGTLLC AASGYPQPNV TWLQCSGHTD RCDEAQVLQV WDDPYPEVLS QEPFHKVTVQ SLLTVETLEH NQTYECRAHN SVGSGSWAFI PISAGAHTHP PDE |
| 13 | Human CSF-1R UniProt P07333 | MGPGVLLLLL VATAWHGQGI PVIEPSVPEL VVKPGATVTL RCVGNGSVEW DGPPSPHWTL YSDGSSSILS TNNATFQNTG TYRCTEPGDP LGGSAAIHLY VKDPARPWNV LAQEVVVFED QDALLPCLLT DPVLEAGVSL VRVRGRPLMR HTNYSFSPWH GFTIHRAKFI QSQDYQCSAL MGGRKVMSIS IRLKVQKVIP GPPALTLVPA ELVRIRGEAA QIVCSASSVD VNFDVFLQHN NTKLAIPQQS DFHNNRYQKV LTLNLDQVDF QHAGNYSCVA SNVQGKHSTS MFFRVVESAY LNLSSEQNLI QEVTVGEGLN LKVMVEAYPG LQGFNWTYLG PFSDHQPEPK LANATTKDTY RHTFTLSLPR LKPSEAGRYS FLARNPGGWR ALTFELTLRY PPEVSVIWTF INGSGTLLCA ASGYPQPNVT WLQCSGHTDR CDEAQVLQVW DDPYPEVLSQ EPFHKVTVQS LLTVETLEHN QTYECRAHNS VGSGSWAFIP ISAGAHTHPP DEFLFTPVVV ACMSIMALLL LLLLLLLYKY KQKPKYQVRW KIIESYEGNS YTFIDPTQLP YNEKWEFPRN NLQFGKTLGA GAFGKVVEAT AFGLGKEDAV LKVAVKMLKS TAHADEKEAL MSELKIMSHL GQHENIVNLL GACTHGGPVL VITEYCCYGD LLNFLRRKAE AMLGPSLSPG QDPEGGVDYK NIHLEKKYVR RDSGFSSQGV DTYVEMRPVS TSSNDSFSEQ DLDKEDGRPL ELRDLLHFSS QVAQGMAFLA SKNCIHRDVA ARNVLLTNGH VAKIGDFGLA RDIMNDSNYI VKGNARLPVK WMAPESIFDC VYTVQSDVWS YGILLWEIFS LGLNPYPGIL VNSKFYKLVK DGYQMAQPAF APKNIYSIMQ ACWALEPTHR PTFQQICSFL QEQAQEDRRE RDYTNLPSSS RSGGSGSSSS ELEEESSSEH LTCCEQGDIA QPLLQPNNYQ FC |

**Examples**

Liquid drug product formulations for intravenous (i.v.) administration according to the invention were developed as follows.

Example 1: Preparation of liquid formulations for Initial Formulation Screening

The huMAb CSF-1R liquid formulations F1 to F16 as listed in Table 1 were prepared at a protein concentration of 50 mg/ml.

Anti-CSF-1R antibody (emactuzumab) was manufactured by techniques generally known from the production of recombinant proteins and as described in WO 2011/070024. For preparing the pharmaceutical formulations in accordance with the examples the antibody was provided at a concentration of approximately 20 mg/mL in a 20 mM histidine buffer (a L-histidine/HCl buffer) at a pH of approximately 5.5. The CSF-1R antibody used in the examples is a humanized antibody comprising heavy chains comprising the amino acid sequence of SEQ ID NO:9 and light chains comprising the amino acid sequence of SEQ ID NO:10.

The excipients of the formulation in accordance with the present invention are widely used in the practice and known to the person skilled in the art. There is therefore no need to explain them here in detail.

For the preparation of the liquid formulations anti-CSF-1R antibody was buffer-exchanged against a diafiltration buffer containing the anticipated buffer composition and concentrated by ultrafiltration to an antibody concentration of approximately 80 mg/mL. After completion of the ultrafiltration operation, the excipients (e.g. sucrose, methionine) were added as stock solutions to the antibody solution. The surfactant was then added as a 125-fold stock solution. Finally, the protein concentration was adjusted with a buffer to the final anti-CSF-1R antibody concentration of approximately 50 mg/mL.

All formulations were sterile-filtered through 0.22 $\mu$m low protein binding filters and aseptically filled into sterile 6 mL glass vials closed with ETFE (Copolymer of ethylene and tetrafluoroethylene)-coated rubber stoppers and alucrimp caps. The fill volume was approx. 2.4 mL. These formulations were stored at different ICH climate conditions (5°C, 25°C and 40°C) for different intervals of time and stressed by shaking (1 week at a shaking frequency of 200 min$^{-1}$ at 5°C and 25°C) and freeze-thaw stress methods (five cycles at -80°C/+5°C).

Table 1 - Overview of the different formulations

| | Buffer | pH | Protein Conc | PS20 (% w/v) | P188 (% w/v) | Methionine (mM) | Sucrose (mM) | Trehalose (mM) | NaCL (mM) | Fill volume (mL) |
|---|---|---|---|---|---|---|---|---|---|---|---|
| F1 | 20mM Histidine-HCl | 6.0 | 50 mg/mL | 0.04 | | | 220 | | | 2.7 |
| F2 | | | | 0.04 | | 10 | 220 | | | |
| F3 | | | | | 0.04 | | 220 | | | |
| F4* | | | | 0.04 | | | | | 130 | |
| F5* | | | | 0.04 | | 10 | | | 130 | |
| F6* | | | | | 0.04 | | | | 130 | |
| F7 | 20 mM Histidine-Acetate | 5.5 | | 0.04 | | | 220 | | | |
| F8 | | | | 0.04 | | 10 | 220 | | | |
| F9 | | | | | 0.04 | | 220 | | | |
| F10* | | | | 0.04 | | | | | 130 | |
| F11* | | | | 0.04 | | 10 | | | 130 | |
| F12* | | | | | 0.04 | | | | 130 | |
| F13 | 20 mM Histidine-HCl | 6.0 | 25 mg/mL | 0.02 | | | | 240 | | |
| F14 | | | 50 mg/ml | 0.04 | | | | 240 | | |
| F15 | | | | 0.04 | | 10 | | 240 | | |
| F16 | | | | | 0.04 | | | 240 | | |

Formulations marked with * are reference examples.

The samples were analyzed before and after applying the stress tests by the following analytical methods

- UV spectrophotometry

- Size Exclusion Chromatography (SEC)

- Ion exchange chromatography (IEC)

- Clarity and opalescence of the solution

- Analytical Protein A Chromatography

- Visual inspection

UV spectroscopy, used for determination of protein content, was performed on a Perkin Elmer λ35 UV spectrophotometer in a wavelength range from 240 nm to 400 nm. Neat protein samples were diluted to approx. 0.5 mg/mL with the corresponding formulation buffer. The protein concentration was calculated according to equation 1.

$$\text{Equation 1:} \quad \text{Protein content} = \frac{A(280) - A(320) \times dil.\,factor}{\varepsilon \left\langle \frac{cm^2}{mg} \right\rangle \times d \langle cm \rangle}$$

The UV light absorption at 280 nm was corrected for light scattering at 320 nm and multiplied with the dilution factor, which was determined from the weighed masses and densities of the neat sample and the dilution buffer. The numerator was divided by the product of the cuvette's path length d and the extinction coefficient ε.

Size Exclusion Chromatography (SEC) was used to detect soluble high molecular weight species (aggregates) and low molecular weight hydrolysis products (LMW) in the formulations. The method was performed on a Waters Alliance 2695 HPLC instrument with a Waters W2487 Dual Absorbance Detector and equipped with a Waters BioSuite 250 column. Intact monomer, aggregates and hydrolysis products were separated by an isocratic elution profile, using 0.2M $K_2HPO_4$ / 0.25M KCL, pH 7.0 as mobile phase, and were detected at a wavelength of 280 nm.

Ion Exchange Chromatography (IEC) was performed to detect chemical degradation products altering the net charge of the antibody in the formulations. The method used a Waters Alliance 2695 HPLC instrument with a Waters W2487 Dual Absorbance Detector and equipped (detection wavelength 280nm) and a MabPac SCX-10, 4mm x 150mm column. 5mM MES, 15mM NaCl, pH 6.5 and 5mM BICINE, 30mM NaCl, pH 8.5 and 5mM BICINE, 1M NaCl, pH 8.5 are used as mobile phases A, B and C, respectively, at a flow rate of 0.5 mL/min.

Gradient program:

| Time | %A | %B | %C |
|------|-----|-----|-----|
| 0.0 | 100 | 0 | 0 |
| 5.0 | 100 | 0 | 0 |
| 50.0 | 0 | 100 | 0 |
| 54.0 | 0 | 100 | 0 |
| 55.0 | 100 | 0 | 0 |
| 65.0 | 100 | 0 | 0 |
| 80.0 | 0 | 0 | 100 |
| 85.0 | 0 | 0 | 100 |
| 86.0 | 100 | 0 | 0 |
| 96.0 | 100 | 0 | 0 |

Clarity and the degree of opalescence were measured as Formazine Turbidity Units (FTU) by the method of nephelometry. The neat sample was transferred into a 11 mm diameter clearglass tube and placed into a HACH 2100AN turbidimeter.

Analytical Protein A chromatography was performed to monitor the oxidation status of the four conserved methionine side chains in the Fc part of the anti-CSF-1R antibody. The method was performed on a Waters Alliance 2695 HPLC instrument with a Waters W2487 Dual Absorbance Detector (detection wavelength 280 nm), equipped with a Poros A720 4.6 mm x 50 mm column from Applied Biosystems, USA. PBS from Gibco, Invitrogen and and 0.1M acetic acid, 0.15M sodium chloride, pH 2.8 were used as mobile phases A and B, respectively, at a flow rate of 2.0 mL/min:

Gradient program:

|  | Time (min) | % Mobile Phase A | % Mobile Phase B |
|---|------------|------------------|------------------|
| 1 | 0.01 | 100 | 0 |
| 2 | 10 | 100 | 0 |
| 3 | 40 | 40 | 60 |
| 4 | 41 | 0 | 100 |
| 5 | 51 | 0 | 100 |
| 6 | 52 | 100 | 0 |
| 7 | 62 | 100 | 0 |

Samples were inspected for the presence of visible particles by using a Simplex Ampoule Testing Apparatus OPTIMA 1.

The results of the stability testing for the Formulations F1 to F16 are provided in the tables below. Formulation F2 was determined to be most favorable for obtaining maximum antibody stability and antibody formulations free from particles.

Example 1: Compositions and stability data of liquid anti-CSF-1R antibody formulations

F1 is a liquid formulation with the composition 50 mg/mL anti-CSF-1R antibody, 20 mM Histidine HCl, 220 mM Sucrose, 0.04% Polysorbate 20, at pH 6.0

| Storage condition | Storage Time | Protein conc. (mg/mL) | Size Exclusion-HPLC | | | Ion Exchange-HPLC | | | Turbidity (FTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| - | Initial | 45.2 | 0.7 | 99.2 | 0.1 | 72.13 | 24.5 | 3.38 | 10 | Free from Particles |
| Shaking 5°C | 1 week | 44.7 | 0.8 | 98.7 | 0.5 | | | | | Free from Particles |
| Shaking 25°C | 1 week | 45 | 0.8 | 98.6 | 0.6 | | | | | Free from Particles |
| Freeze/thaw | 4 cycles | 44.5 | 0.8 | 98.7 | 0.5 | | | | | Free from Particles |
| 5°C | 6 months | 45.7 | 1.0 | 98.9 | 0.1 | 72.11 | 23.63 | 4.26 | 9.3 | Free from Particles |
| 25°C | 6months | 45.2 | 1.5 | 96.4 | 2.2 | 61.52 | 34.18 | 4.30 | 9.5 | Free from Particles |

| Storage condition | Storage Time | Analytical Protein A Chromatography |
|---|---|---|
| | | Fc Methionine Oxidation (%) |
| - | Initial | 4.2 |
| 5°C | 6months | 4.5 |
| 25°C | 6 months | 6.2 |

F2 is a liquid formulation with the composition 50 mg/mL anti-CSF-1R antibody, 20 mM Histidine HCl, 220 mM Sucrose, 0.04% Polysorbate 20, 10mM Methionine,
at pH 6.0

| Storage condition | Storage Time | Protein conc. (mg/mL) | Size Exclusion-HPLC | | | Ion Exchange-HPLC | | | Turbidity (FTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| - | Initial | 45.4 | 0.7 | 99.3 | 0.1 | 71.1 | 25.2 | 3.7 | 9.7 | Free from Particles |
| Shaking 5°C | 1 week | 45.0 | 0.8 | 98.8 | 0.5 | | | | 10.2 | Free from Particles |
| Shaking 25°C | 1 week | 45.3 | 0.8 | 98.7 | 0.6 | | | | 11.1 | Free from Particles |
| Freeze/thaw | 4 cycles | 45.4 | 0.8 | 98.7 | 0.5 | | | | 9.7 | Free from Particles |

(continued)

| Storage condition | Storage Time | Protein conc. (mg/mL) | Size Exclusion-HPLC | | | Ion Exchange-HPLC | | | Turbidity (FTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| 5°C | 6 months | 45.9 | 0.9 | 99.0 | 0.1 | 71.9 | 23.7 | 4.4 | 9.3 | Free from Particles |
| 25°C | 6 months | 45.5 | 1.1 | 96.7 | 2.2 | 62.72 | 32.72 | 4.6 | 9.6 | Free from Particles |
| 5°C | 9 months | 49.3 | 0.8 | 98.5 | 0.7 | 71.8 | 23.2 | 5.1 | 10.4 | Free from Particles |
| 25°C | 9 months | 49.1 | 1.1 | 96.8 | 2.1 | 58.4 | 36.4 | 5.2 | 8.9 | Free from Particles |
| 5°C | 12 months | 49.7 | 0.8 | 99.1 | 0.1 | 71.6 | 23.4 | 5.1 | 9.7 | Free from Particles |
| 25°C | 12 months | 49.1 | 1.1 | 96.1 | 2.8 | 54.5 | 40.8 | 4.8 | 9.7 | Free from Particles |
| 5°C | 24 months | 52.2 | 0.8 | 98.2 | 0.9 | 71.4 | 23.9 | 4.7 | 9.7 | Free from Particles |
| -20°C* | 24 months | 52.1 | 0.7 | 99.3 | 0.0 | 73.1 | 22.2 | 4.8 | 9.7 | |
| -40°C* | 24 months | 51.9 | 0.7 | 99.3 | 0.0 | 73.6 | 22.1 | 4.3. | 9.8 | |
| **\* in stainless steel container** | | | | | | | | | | |

| Storage condition | Storage Time | Analytical Protein A Chromatography |
|---|---|---|
| | | Fc Methionine Oxidation (%) |
| - | Initial | 4.0 |
| 5°C | 6 months | 3.9 |
| 25°C | 6 months | 3.9 |

| Storage condition | Storage Time | Methionine content (mM) | PS20 content (mg/mL | Potency |
|---|---|---|---|---|
| - | Initial | | 0.41 | |
| 5°C | 24 months | 10 | 0.33 | 99% |
| -20°C* | 24 months | 11.1 | 0.38 | 100% |
| -40°C* | 24 months | 10.5 | 0.37 | 98% |
| **\* in stainless steel container** | | | | |

**F3** is a liquid formulation with the composition 50 mg/mL anti-CSF-1R antibody, 20 mM Histidine HCl, 220 mM Sucrose, 0.04% Poloxamer 188, at pH 6.0

| Storage condition | Storage Time | Protein conc. (mg/mL) | Size Exclusion-HPLC | | | Ion Exchange-HPLC | | | Turbidity (FTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| - | Initial | 45.4 | 0.7 | 99.3 | 0.1 | 71.4 | 25.1 | 3.5 | 9.7 | Free from Particles |
| Shaking 5°C | 1 week | 45.9 | 0.8 | 98.7 | 0.5 | | | | 10.1 | Free from Particles |
| Shaking 25°C | 1 week | 45.2 | 0.8 | 98.6 | 0.6 | | | | 10.8 | Free from Particles |
| Freeze/thaw | 4 cycles | 45.3 | 0.8 | 98.7 | 0.5 | | | | 10.0 | Free from Particles |
| 5°C | 6 months | 45.9 | 1.0 | 98.9 | 0.1 | 72.16 | 23.7 | 4.1 | 9.3 | Free from Particles |
| 25°C | 6 months | 45.3 | 1.5 | 96.3 | 2.2 | 61.68 | 34.0 | 4.3 | 9.7 | Free from Particles |

| Storage condition | Storage Time | Analytical Protein A Chromatography |
|---|---|---|
| | | Fc Methionine Oxidation (%) |
| - | Initial | 3.8 |
| 5°C | 6 months | 5.0 |
| 25°C | 6 months | 6.3 |

F4 is a liquid formulation with the composition 50 mg/mL anti-CSF-1R antibody, 20 mM Histidine HCl, 130 mM NaCl, 0.04% Polysorbate 20, at pH 6.0 (Reference Example)

| Storage condition | Storage Time | Protein conc. (mg/mL) | Size Exclusion-HPLC | | | Ion Exchange-HPLC | | | Turbidity (FTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| - | Initial | 47.8 | 0.7 | 99.2 | 0.1 | 71.2 | 25.2 | 3.6 | 22.7 | Free from Particles |
| Shaking 5°C | 1 week | 47.6 | 0.8 | 98.7 | 0.5 | | | | 23.1 | Free from Particles |
| Shaking 25°C | 1 week | 47.3 | 0.9 | 98.6 | 0.6 | | | | 23.7 | Free from Particles |
| Freeze/thaw | 5 cycles | 47.4 | 0.8 | 98.7 | 0.5 | | | | 9.8 | Free from Particles |
| 5°C | 6 months | 47.6 | 1.1 | 98.8 | 0.1 | 72.3 | 23.4 | 4.2 | 23.2 | With many particles |
| 25°C | 6 months | 47.0 | 1.6 | 96.3 | 02.2 | 63.1 | 32.4 | 4.5 | 24.9 | With many particles |

| Storage condition | Storage Time | Analytical Protein A Chromatography |
|---|---|---|
| | | Fc Methionine Oxidation (%) |
| - | Initial | 3.9 |
| 5°C | 6 months | 5.1 |
| 25°C | 6 months | 6.0 |

F5 is a liquid formulation with the composition 50 mg/mL anti-CSF-1R antibody, 20 mM Histidine HCl, 130 mM NaCl, 0.04% Polysorbate 20, 10mM Methionine,
at pH 6.0 (Reference Example)

| Storage condition | Storage Time | Protein conc. (mg/mL) | Size Exclusion-HPLC | | | Ion Exchange-HPLC | | | Turbidity (FTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| - | Initial | 47.6 | 0.7 | 99.3 | 0.1 | 71.6 | 24.8 | 3.8 | 23.1 | Free from Particles |
| Shaking 5°C | 1 week | 47.8 | 0.8 | 98.7 | 0.5 | | | | 23.2 | Free from Particles |
| Shaking 25°C | 1 week | 47.1 | 0.8 | 98.7 | 0.5 | | | | 23.4 | Free from Particles |
| Freeze/thaw | 5 cycles | 47.6 | 0.8 | 98.7 | 0.5 | | | | 23.3 | Free from Particles |
| 5°C | 6 months | 47.4 | 1.0 | 99.0 | 0.1 | 71.6 | 23.0 | 5.3 | 22.8 | With many particles |
| 25°C | 6 months | 47.8 | 1.2 | 96.6 | 2.1 | 63.7 | 31.7 | 4.6 | 22.8 | With many particles |

| Storage condition | Storage Time | Analytical Protein A Chromatography |
|---|---|---|
| | | Fc Methionine Oxidation (%) |
| - | Initial | 3.8 |
| 5°C | 6 months | 3.6 |
| 25°C | 6 months | 4.3 |

**F6** is a liquid formulation with the composition 50 mg/mL anti-CSF-1R antibody, 20 mM Histidine HCl, 130 mM NaCl, 0.04% Poloxamer 188, at pH 6.0 (Reference Example)

| Storage condition | Storage Time | Protein conc. (mg/mL) | Size Exclusion-HPLC | | | Ion Exchange-HPLC | | | Turbidity (FTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| - | Initial | 47.4 | 0.8 | 99.2 | 0.1 | 71.6 | 24.8 | 3.6 | 23.8 | Free from Particles |
| Shaking 5°C | 1 week | 47.5 | 0.8 | 98.7 | 0.5 | | | | 36.6 | Free from Particles |

(continued)

| Storage condition | Storage Time | Protein conc. (mg/mL) | Size Exclusion-HPLC | | | Ion Exchange-HPLC | | | Turbidity (FTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| Shaking 25°C | 1 week | 47.4 | 0.9 | 98.6 | 0.6 | | | | 23.9 | Free from Particles |
| Freeze/thaw | 5 cycles | 47.1 | 0.8 | 98.7 | 0.5 | | | | 22.8 | Free from Particles |
| 5°C | 6 months | 47.6 | 1.1 | 98.8 | 0.1 | 71.9 | 23.6 | 4.5 | 23.6 | With many particles |
| 25°C | 6 months | 47.3 | 1.6 | 96.3 | 2.1 | 63.2 | 32.4 | 4.5 | 23.5 | With many particles |

| Storage condition | Storage Time | Analytical Protein A Chromatography |
|---|---|---|
| | | Fc Methionine Oxidation (%) |
| - | Initial | 3.8 |
| 5°C | 6 months | 4.9 |
| 25°C | 6 months | 5.9 |

**F7** is a liquid formulation with the composition 50 mg/mL anti-CSF-1R antibody, 20 mM Histidine acetate, 220mM Sucrose, 0.04% Polysorbate 20, at pH 5.5

| Storage condition | Storage Time | Protein conc. (mg/mL) | Size Exclusion-HPLC | | | Ion Exchange-HPLC | | | Turbidity (FTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| - | Initial | 46.3 | 0.7 | 99.3 | 0.1 | 71.8 | 24.5 | 3.7 | 10.0 | Free from Particles |
| Shaking 5°C | 1 week | 46.2 | 0.8 | 98.7 | 0.5 | | | | | Free from Particles |
| Shaking 25°C | 1 week | 46 | 0.8 | 98.6 | 0.5 | | | | | Free from Particles |
| Freeze/thaw | 5 cycles | 46 | 0.8 | 98.7 | 0.5 | | | | | Free from Particles |
| 5°C | 6 months | 45.8 | 1.0 | 98.9 | 0.1 | 72.1 | 23.19 | 4.7 | 10.6 | Essentially Free from Particles |
| 25°C | 6 months | 46.2 | 1.6 | 96.1 | 2.3 | 58.4 | 36.6 | 5.1 | 11.2 | Essentially Free from Particles |

| Storage condition | Storage Time | Analytical Protein A Chromatography |
|---|---|---|
| | | Fc Methionine Oxidation (%) |
| - | Initial | 3.9 |

(continued)

| Storage condition | Storage Time | Analytical Protein A Chromatography |
| --- | --- | --- |
| | | Fc Methionine Oxidation (%) |
| 5°C | 6 months | 4.7 |
| 25°C | 6 months | 6.7 |

**F8** is a liquid formulation with the composition 50 mg/mL anti-CSF-1R antibody, 20 mM Histidine acetate, 220mM Sucrose, 0.04% Polysorbate 20, 10mM Methionine at pH 5.5

| Storage condition | Storage Time | Protein conc. (mg/mL) | Size Exclusion-HPLC | | | Ion Exchange-HPLC | | | Turbidity (FTU) | Visible particles |
| --- | --- | --- | --- | --- | --- | --- | --- | --- | --- | --- |
| | | | HMW (%) | Monomer (%) | LMW (%) | Main Peak (%) | Acidic Species (%) | Basic species (%) | | |
| - | Initial | 46.2 | 0.7 | 99.3 | 0.1 | 71.6 | 24.5 | 3.9 | 12.7 | Free from Particles |
| Shaking 5°C | 1 week | 46.3 | 0.8 | 98.8 | 0.5 | | | | 9.2 | Free from Particles |
| Shaking 25°C | 1 week | 46.1 | 0.8 | 98.7 | 0.5 | | | | 9.4 | Free from Particles |
| Freeze/thaw | 5 cycles | 46.4 | 0.8 | 98.8 | 0.5 | | | | 8.7 | Free from Particles |
| 5°C | 6 months | 46.1 | 0.9 | 99.0 | 0.1 | 72.5 | 23.1 | 4.4 | 8.7 | Free from Particles |
| 25°C | 6 months | 46.0 | 1.2 | 96.5 | 2.3 | 59.6 | 35.5 | 4.8 | 8.1 | Free from Particles |

| Storage condition | Storage Time | Analytical Protein A Chromatography |
| --- | --- | --- |
| | | Fc Methionine Oxidation (%) |
| - | Initial | 3.9 |
| 5°C | 6 months | 4.1 |
| 25°C | 6 months | 4.3 |

**F9** is a liquid formulation with the composition 50 mg/mL anti-CSF-1R antibody, 20 mM Histidine acetate, 220mM Sucrose, 0.04% Poloxamer 188, at pH 5.5

| Storage condition | Storage Time | Protein conc. (mg/mL) | Size Exclusion-HPLC | | | Ion Exchange-HPLC | | | Turbidity (FTU) | Visible particles |
| --- | --- | --- | --- | --- | --- | --- | --- | --- | --- | --- |
| | | | HMW (%) | Monomer (%) | LMW (%) | Main Peak (%) | Acidic Species (%) | Basic species (%) | | |
| - | Initial | 46.2 | 0.7 | 98.3 | 0.1 | 71.0 | 24.6 | 4.4 | 9.0 | Free from Particles |
| Shaking 5°C | 1 week | 46.3 | 0.8 | 98.7 | 0.5 | | | | 9.1 | Free from Particles |
| Shaking 25°C | 1 week | 46.1 | 0.8 | 98.7 | 0.5 | | | | 9.1 | Free from Particles |
| Freeze/thaw | 5 cycles | 45.7 | 0.8 | 98.8 | 0.4 | | | | 9.0 | Free from Particles |

(continued)

| Storage condition | Storage Time | Protein conc. (mg/mL) | Size Exclusion-HPLC | | | Ion Exchange-HPLC | | | Turbidity (FTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| 5°C | 6 months | 46.2 | 1.0 | 98.9 | 1.0 | 72.13 | 23.22 | 4.7 | 8.7 | Free from Particles |
| 25°C | 6 months | 46.0 | 1.6 | 96.1 | 1.6 | 58.3 | 36.6 | 5.1 | 9.2 | Free from Particles |

| Storage condition | Storage Time | Analytical Protein A Chromatography |
|---|---|---|
| | | Fc Methionine Oxidation (%) |
| - | Initial | 3.8 |
| 5°C | 6 months | 4.4 |
| 25°C | 6 months | 6.8 |

**F10** is a liquid formulation with the composition 50 mg/mL anti- CSF-1R antibody, 20 mM Histidine acetate, 130 mM NaCl, 0.04% Polysorbate 20, at pH 5.5 (Reference Example)

| Storage condition | Storage Time | Protein conc. (mg/mL) | Size Exclusion-HPLC | | | Ion Exchange-HPLC | | | Turbidity (FTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| - | Initial | 48.5 | 0.7 | 99.2 | 0.1 | 71.2 | 24.7 | 4.2 | 24.4 | Free from Particles |
| Shaking 5°C | 1 week | 48.5 | 0.8 | 98.7 | 0.5 | | | | 24.6 | Free from Particles |
| Shaking 25°C | 1 week | 48.3 | 0.8 | 98.6 | 0.5 | | | | 24.9 | Free from Particles |
| Freeze/thaw | 5 cycles | 48.3 | 0.8 | 98.8 | 0.5 | | | | 23.7 | Free from Particles |
| 5°C | 6 months | 48.2 | 1.2 | 98.7 | 0.1 | 72.4 | 23.1 | 4.5 | 24.4 | With many particles |
| 25°C | 6 months | 48.0 | 1.9 | 95.7 | 2.4 | 60.3 | 34.1 | 5.6 | 24.2 | With many particles |

| Storage condition | Storage Time | Analytical Protein A Chromatography |
|---|---|---|
| | | Fc Methionine Oxidation (%) |
| - | Initial | 3.7 |
| 5°C | 6 months | 4.7 |
| 25°C | 6 months | 5.8 |

**F11** is a liquid formulation with the composition 50 mg/mL anti-CSF-1R antibody, 20 mM Histidine acetate, 130 mM NaCl, 0.04% Polysorbate 20, 10mM methionine

at pH 5.5 (Reference Example)

| Storage condition | Storage Time | Protein conc. (mg/mL) | Size Exclusion-HPLC | | | Ion Exchange-HPLC | | | Turbidity (FTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| - | Initial | 48.4 | 0.7 | 99.3 | 0.1 | 71.4 | 24.5 | 4.2 | 23.7 | Free from Particles |
| Shaking 5°C | 1 week | 48.7 | 0.7 | 98.8 | 0.5 | | | | 23.7 | Free from Particles |
| Shaking 25°C | 1 week | 48.2 | 0.8 | 98.7 | 0.6 | | | | 23.5 | Free from Particles |
| Freeze/thaw | 5 cycles | 48.3 | 0.8 | 98.87 | 0.5 | | | | 23.4 | Free from Particles |
| 5°C | 6 months | 48.6 | 1.0 | 98.93 | 0.1 | 72.6 | 22.6 | 4.8 | 23.7 | With many particles |
| 25°C | 6 months | 48.2 | 1.4 | 96.1 | 2.4 | 60.9 | 33.6 | 5.5 | 24.0 | With many particles |

| Storage condition | Storage Time | Analytical Protein A Chromatography |
|---|---|---|
| | | Fc Methionine Oxidation (%) |
| - | Initial | 4.0 |
| 5°C | 9 months | 4.0 |
| 25°C | 9 months | 4.3 |

**F12** is a liquid formulation with the composition 50 mg/mL anti-CSF-1R antibody, 20 mM Histidine acetate, 130 mM NaCl, 0.04% Poloxamer 188, at pH 5.5 (Reference Example)

| Storage condition | Storage Time | Protein conc. (mg/mL) | Size Exclusion-HPLC | | | Ion Exchange-HPLC | | | Turbidity (FTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| - | Initial | 48.3 | 0.7 | 99.2 | 0.1 | 71.6 | 24.4 | 4.0 | 24.1 | Free from Particles |
| Shaking 5°C | 1 week | 48.5 | 0.8 | 98.6 | 0.54 | | | | 24.9 | Free from Particles |
| Shaking 25°C | 1 week | 48.2 | 0.8 | 98.6 | 0.6 | | | | 25.0 | Free from Particles |
| Freeze/thaw | 5 cycles | 47.9 | 0.8 | 98.7 | 0.5 | | | | 23.7 | Free from Particles |
| 5°C | 6 months | 47.0 | 1.2 | 98.7 | 0.1 | 72.8 | 23.0 | 4.217.9 | 24.8 | With many Particles |

(continued)

| Storage condition | Storage Time | Protein conc. (mg/mL) | Size Exclusion-HPLC | | | Ion Exchange-HPLC | | | Turbidity (FTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| 25°C | 6 months | 48.2 | 1.95 | 95.7 | 2.4 | 60.6 | 34.0 | 5.4 | 25.2 | With many Particles |

| Storage condition | Storage Time | Analytical Protein A Chromatography |
|---|---|---|
| | | Fc Methionine Oxidation (%) |
| - | Initial | 4.1 |
| 5°C | 6 months | 4.7 |
| 25°C | 6 months | 6.352 |

**F13** is a liquid formulation with the composition 25 mg/mL anti-CSF-1R antibody, 20 mM Histidine HCl, 240 mM Trehalose, 0.02% Polysorbate 20, at pH 6.0

| Storage condition | Storage Time | Protein conc. (mg/mL) | Size Exclusion-HPLC | | | Ion Exchange-HPLC | | | Turbidity (FTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| - | Initial | 23.1 | 0.6 | 99.3 | 0.1 | 71.1 | 25.2 | 3.7 | 7.1 | Free from Particles |
| Shaking 5°C | 1 week | 22.9 | 0.7 | 98.7 | 0.5 | | | | 7.6 | Free from Particles |
| Shaking 25°C | 1 week | 22.8 | 0.7 | 98.7 | 0.6 | | | | 7.5 | Free from Particles |
| Freeze/thaw | 5 cycles | 22.9 | 0.7 | 98.8 | 0.5 | | | | 7.1 | Free from Particles |
| 5°C | 6 months | 22.9 | 0.9 | 99.0 | 0.1 | 72.3 | 23.7 | 4.0 | 7.1 | Free from Particles |
| 25°C | 6 months | 22.8 | 1.1 | 96.7 | 2.2 | 61.8 | 33.9 | 4.3 | 7.1 | Free from Particles |

| Storage condition | Storage Time | Analytical Protein A Chromatography |
|---|---|---|
| | | Fc Methionine Oxidation (%) |
| - | Initial | 3.6 |
| 5°C | 6 months | 4.6 |
| 25°C | 6 months | 5.8 |

**F14** is a liquid formulation with the composition 50 mg/mL anti-CSF-1R antibody, 20 mM Histidine HCl, 240 mM Trehalose, 0.04% Polysorbate 20, at pH 6.0

| Storage condition | Storage Time | Protein conc. (mg/mL) | Size Exclusion-HPLC | | | Ion Exchange-HPLC | | | Turbidity (FTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| - | Initial | 45.5 | 0.7 | 99.3 | 0.1 | 72.0 | 24.7 | 3.4 | 9.7 | Free from Particles |
| Shaking 5°C | 1 week | 45.1 | 0.8 | 98.7 | 0.5 | | | | 9.9 | Free from Particles |
| Shaking 25°C | 1 week | 45.0 | 0.8 | 98.6 | 0.6 | | | | 10.2 | Free from Particles |
| Freeze/thaw | 5 cycles | 45.1 | 0.8 | 98.7 | 0.5 | | | | 10.0 | Free from Particles |
| 5°C | 6 months | 45.1 | 1.0 | 98.9 | 0.1 | 72.3 | 23.6 | 4.1 | 9.6 | Free from Particles |
| 25°C | 6 months | 44.9 | 1.5 | 96.4 | 2.2 | 62.0 | 33.7 | 4.3 | 9.5 | Free from Particles |

| Storage condition | Storage Time | Analytical Protein A Chromatography |
|---|---|---|
| | | Fc Methionine Oxidation (%) |
| - | Initial | 2.1 |
| 5°C | 6 months | 4.2 |
| 25°C | 6 months | 5.1 |

**F15** is a liquid formulation with the composition 50 mg/mL anti-CSF-1R antibody, 20 mM Histidine HCl, 240 mM Trehalose, 0.04% Polysorbate 20, 10mM Methionine at pH 6.0

| Storage condition | Storage Time | Protein conc. (mg/mL) | Size Exclusion-HPLC | | | Ion Exchange-HPLC | | | Turbidity (FTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| - | Initial | 45.4 | 0.7 | 99.3 | 0.1 | 71.6 | 24.8 | 3.7 | 9.3 | Free from Particles |
| Shaking 5°C | 1 week | 44.9 | 0.8 | 98.7 | 0.5 | | | | 10.0 | Free from Particles |
| Shaking 25°C | 1 week | 44.9 | 0.8 | 98.6 | 0.6 | | | | 10.51.1 | Essentially free from Particles |
| Freeze/thaw | 5 cycles | 45.2 | 0.8 | 98.87 | 0.5 | | | | 9.8 | Free from Particles |
| 5°C | 6 months | 45.0 | 0.9 | 99.0 | 0.1 | 72.7 | 23.3 | 4.0 | 9.5 | Essentially free from Particles |
| 25°C | 6 months | 45.1 | 1.2 | 96.7 | 2.1 | 62.7 | 32.6 | 4.7 | 9.3 | Free from Particles |

| Storage condition | Storage Time | Analytical Protein A Chromatography |
| --- | --- | --- |
| | | Fc Methionine Oxidation (%) |
| - | Initial | 3.7 |
| 5°C | 6 months | 3.6 |
| 25°C | 6 months | 4.5 |

**F16** is a liquid formulation with the composition 50 mg/mL anti-CSF-1R antibody, 20 mM Histidine HCl, 240 mM Trehalose, 0.04% Poloxamer 188, at pH 6.0

| Storage condition | Storage Time | Protein conc. (mg/mL) | Size Exclusion-HPLC | | | Ion Exchange-HPLC | | | Turbidity (FTU) | Visible particles |
| --- | --- | --- | --- | --- | --- | --- | --- | --- | --- | --- |
| | | | HMW (%) | Monomer (%) | LMW (%) | Main Peak (%) | Acidic Species (%) | Basic species (%) | | |
| - | Initial | 45.3 | 0.7 | 99.2 | 0.1 | 71.2 | 24.7 | 4.1 | 9.6 | Free from Particles |
| Shaking 5°C | 1 week | 45.0 | 0.8 | 98.7 | 0.5 | | | | 10.1 | Free from Particles |
| Shaking 25°C | 1 week | 45.1 | 0.8 | 98.7 | 0.5 | | | | 10.1 | Free from Particles |
| Freeze/thaw | 5 cycles | 44.6 | 0.8 | 98.7 | 0.5 | | | | 10.0 | Free from Particles |
| 5°C | 6 months | 45.1 | 1.0 | 98.9 | 0.1 | 72.4 | 23.7 | 3.9 | 9.4 | Free from Particles |
| 25°C | 6 months | 45.4 | 1.5 | 96.4 | 2.2 | 61.8 | 33.7 | 18.6 | 9.7 | Essentially free from Particles |

| Storage condition | Storage Time | Analytical Protein A Chromatography |
| --- | --- | --- |
| | | Fc Methionine Oxidation (%) |
| - | Initial | 3.8 |
| 5°C | 6 months | 4.7 |
| 25°C | 6 months | 6.4 |

**Example 2: Compositions and stability data of liquid anti-CSF-1R antibody drug products comprising formulation F2 in vials**

Two drug products based on Formulation F2 were manufactured and demonstrated good stability. Drug Product 1 contains 9.0mL formulation F2 in a 20 ml glass vial, and Drug Product 2 contains 46.4 mL formulation F2 in a 50 ml glass vial.

**Drug Product 1 (F2) stability data**

| Storage condition | Storage Time | Protein conc. (mg/mL) | Size Exclusion-HPLC | | | Ion Exchange-HPLC | | | Turbidity (NTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| - | Initial | 51.6 | 0.9 | 99.1 | 0.0 | 70.1 | 24.4 | 5.5 | 8.6 | Free from Particles |
| 40°C+75% r.h. | 1 weeek | 52.1 | 1.1 | 98.8 | 0.1 | 66.6 | 27.6 | 5.9 | 8.9 | Free from Particles |
| 40°C+75% r.h | 2 weeks | 52.0 | 1.2 | 98.6 | 0.2 | 63.1 | 31.1 | 5.9 | 8.9 | Free from Particles |
| 5°C | 1 month | 51.8 | 0.9 | 99.1 | 0.0 | 70.7 | 23.9 | 5.4 | 8.9 | Free from Particles |
| 25°C+60% r.h | 1 month | 52.3 | 1.0 | 98.9 | 0.1 | 69.3 | 25.2 | 5.5 | 8.8 | Free from Particles |
| 40°C+75% r.h | 1 month | 52.3 | 1.3 | 98.3 | 0.4 | 56.2 | 38.1 | 5.6 | 9.2 | Free from Particles |
| 40°C+75% r.h | 6 weeks | 52.0 | 1.5 | 97.9 | 0.6 | 49.0 | 45.9 | 5.1 | 9.2 | Free from Particles |
| 40°C+75% r.h | 8 weeks | 51.8 | 1.7 | 97.5 | 0.8 | 43.2 | 51.8 | 5.0 | 8.9 | Free from Particles |
| 5°C | 3 months | 51.8 | 1.0 | 99.0 | 0.0 | 69.8 | 24.5 | 5.7 | 7.6 | Free from Particles |
| 25°C | 3 months | 51.8 | 1.2 | 98.6 | 0.2 | 64.4 | 30.1 | 5.5 | 8.3 | Free from Particles |
| 5°C | 6 months | 51.6 | 1.0 | 98.9 | 0.0 | 70.0 | 24.9 | 5.1 | 8.1 | Free from Particles |
| 25°C | 6 months | 51.5 | 1.3 | 98.3 | 0.4 | 58.4 | 36.8 | 4.8 | 8.3 | Free from Particles |
| 5°C | 9 months | 51.3 | 1.1 | 98.8 | 0.1 | 68.3 | 26.1 | 5.6 | 8.2 | Free from Particles |
| 5°C | 12 months | 51.2 | 1.1 | 98.8 | 0.1 | 69.1 | 25.4 | 5.5 | 8.3 | Free from Particles |
| 25°C | 12 months | 51.5 | 1.5 | 97.7 | 0.8 | 47.4 | 48.0 | 4.6 | 8.3 | Free from Particles |

**Drug Product 2 (F2) stability data**

| Storage condition | Storage Time | Protein conc. (mg/mL) | Size Exclusion-HPLC | | | Ion Exchange-HPLC | | | Turbidity (NTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| - | Initial | 50.9 | 0.9 | 99.1 | 0.0 | 70.2 | 24.3 | 5.5 | 9.3 | Free from Particles |
| 40°C+75% r.h. | 1 weeek | 51.0 | 1.1 | 98.8 | 0.1 | 67.2 | 27.0 | 5.8 | 8.9 | Free from Particles |

(continued)

| Storage condition | Storage Time | Protein conc. (mg/mL) | Size Exclusion-HPLC | | | Ion Exchange-HPLC | | | Turbidity (NTU) | Visible particles |
|---|---|---|---|---|---|---|---|---|---|---|
| | | | HMW (%) | Monomer (%) | LMW (%) | Main Peak (%) | Acidic Species (%) | Basic species (%) | | |
| 40°C+75% r.h | 2 weeks | 51.0 | 1.2 | 98.6 | 0.2 | 63.5 | 30.7 | 5.8 | 8.9 | Free from Particles |
| 5°C | 1 month | 52.2 | 0.9 | 99.0 | 0.0 | 70.7 | 23.9 | 5.4 | 8.6 | Free from Particles |
| 25°C+60% r.h | 1 month | 52.3 | 1.1 | 98.8 | 0.1 | 69.4 | 25.1 | 5.5 | 8.8 | Free from Particles |
| 40°C+75% r.h | 1 month | 52.4 | 1.3 | 98.3 | 0.4 | 56.5 | 37.8 | 5.7 | 9.1 | Free from Particles |
| 40°C+75% r.h | 6 weeks | 52.3 | 1.6 | 97.8 | 0.6 | 49.4 | 45.6 | 5.0 | 9.3 | Free from Particles |
| 40°C+75% r.h | 8 weeks | 51.7 | 1.7 | 97.5 | 0.8 | 43.4 | 51.5 | 5.0 | 9.1 | Free from Particles |
| 5°C | 3 months | 51.8 | 1.0 | 98.9 | 0.0 | 70.0 | 24.4 | 5.6 | 8.3 | Free from Particles |
| 25°C | 3 months | 51.6 | 1.2 | 98.6 | 0.2 | 64.5 | 30.0 | 5.5 | 8.4 | Free from Particles |
| 5°C | 6 months | 51.6 | 1.0 | 98.9 | 0.0 | 70.1 | 24.8 | 5.1 | 8.4 | Free from Particles |
| 25°C | 6 months | 51.6 | 1.3 | 98.3 | 0.4 | 58.9 | 36.5 | 4.6 | 8.5 | Free from Particles |
| 5°C | 9 months | 51.6 | 1.1 | 98.8 | 0.1 | 68.3 | 26.1 | 5.6 | 8.3 | Free from Particles |
| 5°C | 12 months | 51.7 | 1.2 | 98.8 | 0.1 | 69.3 | 25.4 | 5.3 | 8.0 | Free from Particles |
| 25°C | 12 months | 51.7 | 1.5 | 97.7 | 0.8 | 47.4 | 47.8 | 4.5 | 8.7 | Free from Particles |

**Claims**

1. A pharmaceutical formulation comprising:

   - 40 mg/ml to 200 mg/ml of an antibody against CSF-1R comprising a heavy chain variable region comprising the amino acid sequence of SEQ ID NO: 7 and a light chain variable region comprising the amino acid sequence of SEQ ID NO:8;
   - 0.01 % w/v to 0.1% w/v of polysorbate;
   - 5 mM to 100 mM of a histidine buffer;
   - 10 mM to 500 mM of at least one stabilizer, wherein the at least one stabilizer is a saccharide;

   at a pH in the range from 4.5 to 7.0.

2. The pharmaceutical formulation according to claim 1, wherein the concentration of the antibody against CSF-1R is in the range of 40 mg/ml to 100 mg/ml, particularly of 50 mg/ml.

3. The pharmaceutical formulation according to claims 1 or 2, wherein the polysorbate is present in a concentration of 0.04 % w/v.

4. The pharmaceutical formulation according to any one of claims 1 to 3, wherein the histidine buffer is a histidine chloride buffer.

5. The pharmaceutical formulation according to any one of claims 1 to 4, wherein the histidine buffer has a concentration in the range of 10 to 30 mM, particularly of 20 mM.

6. The pharmaceutical formulation according to any one of claims 1 to 5, wherein the pH of the formulation is in the range of 5.0 to 6.5, particularly at 6.0.

7. The pharmaceutical formulation according to any one of claims 1 to 6, wherein the at least one stabilizer is sucrose or trehalose.

8. The pharmaceutical formulation according to any one of claims 1 to 7, wherein the at least one stabilizer is sucrose.

9. The pharmaceutical formulation according to any one of claims 1 to 8, wherein the at least one stabilizer is present in a concentration in the range from 140 to 250 mM, particularly in the range from 210 to 230 mM.

10. The pharmaceutical formulation according to claim 7, comprising a first stabilizer being a saccharide, and methionine as a second stabilizer.

11. The pharmaceutical formulation according to claim 10, wherein the first stabilizer is present in a concentration of 120 to 300 mM, and the second stabilizer methionine is present in a concentration of 5 to 25 mM.

12. The pharmaceutical formulation according to any one of claims 1 to 9, which comprises

    40 to 100 mg/ml antibody against CSF-1R;
    20 mM L-histidine;
    0.03% w/v to 0.05% w/v polysorbate 20;
    210 to 230 mM sucrose;
    optionally 5 to 25 mM methionine;
    at a pH of 6.0 $\pm$ 0.5.

13. The pharmaceutical formulation according to any one of claims 1 to 12, which comprises

    50 mg/ml antibody against CSF-1R;
    20 mM L-histidine;
    0.04% w/v polysorbate 20;
    220 mM sucrose;
    10 mM methionine;
    at a pH of 6.0 $\pm$ 0.5.

14. The pharmaceutical formulation according to any one of claims 1 to 13, which is in a liquid form, in a lyophilized form or in a liquid form reconstituted from a lyophilized form.

15. The pharmaceutical formulation according to any one of claims 1 to 14 for use in treating cancer.

16. The pharmaceutical formulation according to any one of claims 1 to 14, or the pharmaceutical formulation for use of claim 15, for use in combination with another therapeutic agent, in particular an agent blocking PD-L1/PD-1 interaction.

17. The pharmaceutical formulation according to any one of claims 1 to 14 for use in treating pigmented villonodular synovitis (PVNS) or tenosynovial giant cell tumors (TGCT).

**Patentansprüche**

1. Pharmazeutische Formulierung, umfassend:

- 40 mg/ml bis 200 mg/ml eines Antikörpers gegen CSF-1R, umfassend eine variable Region der schweren Kette, umfassend die Aminosäuresequenz von SEQ ID NO: 7, und eine variable Region der leichten Kette, umfassend die Aminosäuresequenz von SEQ ID NO: 8;
- 0,01 % Gew./Vol. bis 0,1 % Gew./Vol. Polysorbat;
- 5 mM bis 100 mM eines Histidinpuffers;
- 10 mM bis 500 mM mindestens eines Stabilisators, wobei der mindestens eine Stabilisator ein Saccharid ist;

bei einem pH-Wert im Bereich von 4,5 bis 7,0.

2. Pharmazeutische Formulierung nach Anspruch 1, wobei die Konzentration des Antikörpers gegen CSF-1R im Bereich von 40 mg/ml bis 100 mg/ml, insbesondere bei 50 mg/ml, liegt.

3. Pharmazeutische Formulierung nach Anspruch 1 oder 2, wobei das Polysorbat in einer Konzentration von 0,04 % Gew./Vol. vorliegt.

4. Pharmazeutische Formulierung nach einem der Ansprüche 1 bis 3, wobei der Histidinpuffer ein Histidinchloridpuffer ist.

5. Pharmazeutische Formulierung nach einem der Ansprüche 1 bis 4, wobei der Histidinpuffer eine Konzentration im Bereich von 10 bis 30 mM, insbesondere von 20 mM, aufweist.

6. Pharmazeutische Formulierung nach einem der Ansprüche 1 bis 5, wobei der pH-Wert der Formulierung im Bereich von 5,0 bis 6,5, insbesondere bei 6,0, liegt.

7. **Pharmazeutische** Formulierung nach einem der Ansprüche 1 bis 6, wobei der mindestens eine Stabilisator Saccharose oder Trehalose ist.

8. Pharmazeutische Formulierung nach einem der Ansprüche 1 bis 7, wobei der mindestens eine Stabilisator Saccharose ist.

9. Pharmazeutische Formulierung nach einem der Ansprüche 1 bis 8, wobei der mindestens eine Stabilisator in einer Konzentration im Bereich von 140 bis 250 mM, insbesondere im Bereich von 210 bis 230 mM, vorliegt.

10. Pharmazeutische Formulierung nach Anspruch 7, umfassend einen ersten Stabilisator, der ein Saccharid ist, und Methionin als zweiten Stabilisator.

11. Pharmazeutische Formulierung nach Anspruch 10, wobei der erste Stabilisator in einer Konzentration von 120 bis 300 mM vorliegt und der zweite Stabilisator Methionin in einer Konzentration von 5 bis 25 mM vorliegt.

12. Pharmazeutische Formulierung nach einem der Ansprüche 1 bis 9, die Folgendes umfasst:

40 bis 100 mg/ml Antikörper gegen CSF-1R;
20 mM L-Histidin;
0,03 % Gew./Vol. bis 0,05 % Gew./Vol. Polysorbat 20;
210 bis 230 mM Saccharose;
gegebenenfalls 5 bis 25 mM Methionin;
bei einem pH-Wert von 6,0 $\pm$ 0,5.

13. Pharmazeutische Formulierung nach einem der Ansprüche 1 bis 12, die Folgendes umfasst:

50 mg/ml Antikörper gegen CSF-1R;
20 mM L-Histidin;
0,04 % Gew./Vol. Polysorbat 20;
220 mM Saccharose;
10 mM Methionin;
bei einem pH-Wert von 6,0 $\pm$ 0,5.

14. Pharmazeutische Formulierung nach einem der Ansprüche 1 bis 13, die in einer flüssigen Form, in einer lyophilisierten Form oder in einer aus einer lyophilisierten Form rekonstituierten flüssigen Form vorliegt.

15. Pharmazeutische Formulierung nach einem der Ansprüche 1 bis 14 zur Verwendung bei der Behandlung von Krebs.

16. Pharmazeutische Formulierung nach einem der Ansprüche 1 bis 14 oder pharmazeutische Formulierung zur Verwendung nach Anspruch 15, zur Verwendung in Kombination mit einem anderen therapeutischen Mittel, insbesondere einem Mittel, das die PD-L1/PD-1-Wechselwirkung blockiert.

17. Pharmazeutische Formulierung nach einem der Ansprüche 1 bis 14 zur Verwendung bei der Behandlung von pigmentierter villonodulärer Synovitis (PVNS) oder tenosynovialen Riesenzelltumoren (TGCT).

**Revendications**

1. Formulation pharmaceutique comprenant :

    - 40 mg/ml à 200 mg/ml d'un anticorps dirigé contre CSF-1R comprenant une région variable de chaîne lourde comprenant la séquence d'acides aminés de SEQ ID NO : 7 et une région variable de chaîne légère comprenant la séquence d'acides aminés de SEQ ID NO : 8 ;
    - 0,01 % p/v à 0,1 % p/v de polysorbate ;
    - 5 mM à 100 mM d'un tampon histidine ;
    - 10 mM à 500 mM d'au moins un stabilisant, l'au moins un stabilisant étant un saccharide ;

    à un pH dans la plage allant de 4,5 à 7,0.

2. Formulation pharmaceutique selon la revendication 1, dans laquelle la concentration de l'anticorps dirigé contre CSF-1R est dans la plage de 40 mg/ml à 100 mg/ml, en particulier de 50 mg/ml.

3. Formulation pharmaceutique selon les revendications 1 ou 2, dans laquelle le polysorbate est présent à une concentration de 0,04 % p/v.

4. Formulation pharmaceutique selon l'une quelconque des revendications 1 à 3, dans laquelle le tampon histidine est un tampon chlorure d'histidine.

5. Formulation pharmaceutique selon l'une quelconque des revendications 1 à 4, dans laquelle le tampon histidine a une concentration dans la plage de 10 à 30 mM, en particulier de 20 mM.

6. Formulation pharmaceutique selon l'une quelconque des revendications 1 à 5, dans laquelle le pH de la formulation est dans la plage de 5,0 à 6,5, en particulier à 6,0.

7. Formulation pharmaceutique selon l'une quelconque des revendications 1 à 6, dans laquelle l'au moins un stabilisant est le saccharose ou le tréhalose.

8. Formulation pharmaceutique selon l'une quelconque des revendications 1 à 7, dans laquelle l'au moins un stabilisant est le saccharose.

9. Formulation pharmaceutique selon l'une quelconque des revendications 1 à 8, dans laquelle l'au moins un stabilisant est présent à une concentration dans la plage allant de 140 à 250 mM, en particulier dans la plage allant de 210 à 230 mM.

10. Formulation pharmaceutique selon la revendication 7, comprenant un premier stabilisant qui est un saccharide, et de la méthionine en tant que second stabilisant.

11. Formulation pharmaceutique selon la revendication 10, dans laquelle le premier stabilisant est présent à une concentration de 120 à 300 mM, et le second stabilisant méthionine est présent à une concentration de 5 à 25 mM.

12. Formulation pharmaceutique selon l'une quelconque des revendications 1 à 9, qui comprend

40 à 100 mg/ml d'anticorps dirigé contre CSF-1R ;
20 mM de L-histidine ;
0,03 % p/v à 0,05 % p/v de polysorbate 20 ;
210 à 230 mM de saccharose ;
éventuellement 5 à 25 mM de méthionine ;
à un pH de 6,0 ± 0,5.

13. Formulation pharmaceutique selon l'une quelconque des revendications 1 à 12, qui comprend

50 mg/ml de l'anticorps dirigé contre CSF-1R ;
20 mM de L-histidine ;
0,04 % p/v de polysorbate 20 ;
220 mM de saccharose ;
10 mM de méthionine ;
à un pH de 6,0 ± 0,5.

14. Formulation pharmaceutique selon l'une quelconque des revendications 1 à 13, qui est sous une forme liquide, sous une forme lyophilisée ou sous une forme liquide reconstituée à partir d'une forme lyophilisée.

15. Formulation pharmaceutique selon l'une quelconque des revendications 1 à 14 pour une utilisation dans le traitement d'un cancer.

16. Formulation pharmaceutique selon l'une quelconque des revendications 1 à 14, ou formulation pharmaceutique pour une utilisation selon la revendication 15, pour une utilisation en association avec un autre agent thérapeutique, en particulier un agent bloquant l'interaction PD-L1/PD-1.

17. Formulation pharmaceutique selon l'une quelconque des revendications 1 à 14 pour une utilisation dans le traitement de la synovite villonodulaire pigmentée (SVP) ou des tumeurs ténosynoviales à cellules géantes (TGCT).

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011070024 A1
- WO 2016128318 A1
- WO 2016106180 A1
- WO 2011070024 A

**Non-patent literature cited in the description**

- **COUSSENS, L. et al.** *Nature*, 1986, vol. 320, 277-280
- **SHERR, C.J. et al.** *Cell*, 1985, vol. 41, 665-676
- **ROUSSEL, M.F. et al.** *Nature*, 1987, vol. 325, 549-552
- **LEE, P.S. et al.** *Embo J.*, 1999, vol. 18, 3616-3628
- **LIN, H. et al.** *Science*, 2008, vol. 320, 807-811
- **CASSIER, P. et al.** *Lancet Oncol.*, 2015, vol. 16, 949-956
- **LI, W. et al.** *EMBO Journal*, 1991, vol. 10, 277-288
- **STANLEY, E.R. et al.** *Mol. Reprod. Dev.*, 1997, vol. 46, 4-10
- **WANG, Z. et al.** *Molecular and Cellular Biology*, 1993, vol. 13, 5348-5359
- **YEUNG, Y-G. et al.** *Molecular & Cellular Proteomics*, 2003, vol. 2, 1143-1155
- **PIXLEY, F. J. et al.** *Trends Cell Biol*, 2004, vol. 14, 628-638
- **MANNING, M. C. et al.** Stability of protein pharmaceuticals. *Pharm Res*, 1989, vol. 6 (11), 903-918
- **ZHENG, J. Y.** ; **JANIS, L. J.** Influence of pH, buffer species, and storage temperature on physicochemical stability of a humanized monoclonal antibody LA298. *Int. J. Pharmaceutics*, 2005, vol. 308, 46-51
- **RIECHMANN, L. et al.** *Nature*, 1988, vol. 332, 323-327
- **NEUBERGER, M.S. et al.** *Nature*, 1985, vol. 314, 268-270
- **LILJEBLAD et al.** *Glyco J*, 2000, vol. 17, 323-329
- **HEELEY**. *Endocr Res*, 2002, vol. 28, 217-229
- **KABAT et al.** Sequences of Proteins of Immunological Interest. 1991
- **CHOTHIA** ; **LESK**. *J. Mol. Biol.*, 1987, vol. 196, 901-917
- **MACCALLUM et al.** *J. Mol. Biol.*, 1996, vol. 262, 732-745